# EUROPEAN PATENT APPLICATION

(11) **EP 4 353 718 A1**
(43) Date of publication of application: **17.04.2024**
(21) Application number: 22820241.2
(22) Date of filing: 08.06.2022
(51) Int. Cl.: C07D 403/12, A61K 31/506, A61P 3/00, A61P 13/12

(54) **METHOD FOR PRODUCING DIHYDROPYRIDAZINE-3,5-DIONE DERIVATIVE**

(30) Priority: 08.06.2021 JP 2021096200
(71) Applicant: CHUGAI SEIYAKU KABUSHIKI KAISHA, Tokyo 115-8543 (JP)
(72) Inventor: MURAKATA, Masatoshi, Tokyo 115-8543 (JP); KOMIYA, Shio, Tokyo 115-8543 (JP); HOU, Zengye, Tokyo 115-8543 (JP); ENOMOTO, Taro, Tokyo 115-8543 (JP); TANIDA, Satoshi, Gotemba-shi, Shizuoka 412-8513 (JP); FUKUDA, Hiroshi, Kamakura-shi, Kanagawa 247-8530 (JP)
(74) Representative: Vossius & Partner Patentanwälte Rechtsanwälte mbB
(86) International application number: PCT/JP2022/023068
(87) International publication number: WO 2022/260062

(57) **Abstract**

The purpose of the present invention is to provide an industrially preferred method for producing a *p*-toluenesulfonate of 7-(2,3-difluoro-4-(2-((2-methoxyethyl)(methyl)amino)ethoxy)benzyl)-10-hydroxy-6-methyl-*N*-(4-methyl-2-(6-methylpyrimidin-4-yl)phenyl)-8-oxo-6,7-diazaspiro[4,5]dec-9-ene-9-carboxamide, which enables a target product to be obtained more inexpensively and easily as compared to conventional methods.

The present invention has attained a method which enables a target product to be efficiently obtained by a step of preparing wet powder of a p-toluenesulfonate of 7-(2,3-difluoro-4-(2-((2-methoxyethyl)(methyl)amino)ethoxy)benzyl)-10-hydroxy-6-methyl-*N-*(4-methyl-2-(6-methylpyrimidin-4-yl)phenyl)-8-oxo-6,7-diazaspiro[4,5]dec-9-ene-9-carboxamide, and a step of drying the wet powder.

## Description

### TECHNICAL FIELD

The present invention relates to a method for producing a dihydropyridazine-3,5-dione derivative.

### BACKGROUND ART

Patients having kidney dysfunction such as chronic kidney disease (CKD) and end-stage kidney disease (ESKD) are known to develop hyperphosphatemia as phosphorus is accumulated in the body. Calcification of the blood vessel from hyperphosphatemia may be a cause of dysfunction of the cardiovascular system. In addition, the calcification of the blood vessel leads to excessive secretion of parathyroid hormone, and causes a bone lesion. Thus, hyperphosphatemia may be a factor of worsening the prognosis and QOL of end-stage kidney-failure patients and dialysis patients (Non Patent Literature 1).

CKD is classified into stages 1 to 5 according to the degree of progression (Non Patent Literature 2 and Non Patent Literature 3). The blood phosphorus level in patients with stages 3 and 4 of CKD is related to the prevalence rate and the mortality rate of cardiovascular diseases, and control of the blood phosphorus level in these patients may lead to alleviation or prevention of cardiovascular diseases. Further, earlier control of a load of phosphates on a patient may alleviate and/or prevent progression of a disease condition in a patient with early-stage CDK (Non Patent Literature 4).

For current treatment of hyperphosphatemia, phosphorus adsorbents aimed at suppressing absorption of phosphoric acid in the gastrointestinal tract are used. As the phosphorus adsorbent, non-metallic polymer adsorbents typified by sevelamer hydrochloride, calcium salt preparations typified by precipitated calcium carbonate, and metallic adsorbents typified by lanthanum carbonate are used, and poor medication compliance due to necessity of dosing at several grams a day and side effects caused by accumulation of calcium in the body have been reported. Therefore, there is strong demand for development of a novel therapeutic agent for hyperphosphatemia in which the above-mentioned problems of phosphorus adsorbents are improved (Non Patent Literature 4).

As sodium-dependent phosphate transporters, three families: NaPi-I, NaPi-II and NaPi-III are known. These families are further classified into isotypes, and for the NaPi-II family, NaPi-IIa, NaPi-IIb and NaPi-IIc are known. In particular, phosphate transporters such as NaPi-IIb, PiT-1 and PiT-2 are known to perform phosphate absorption in the gastrointestinal tract, and selective inhibition of only these phosphate transporters involved in phosphate absorption can expected to produce a strong effect of inhibiting phosphate absorption in the gastrointestinal tract, resulting in reduction of the blood phosphorus level (Non Patent Literature 5, Non Patent Literature 6, Non Patent Literature 7 and Non Patent Literature 8).

NTX 1942 (Patent Literature 1) and condensed thiophene derivatives (Patent Literature 2, Patent Literature 3, Patent Literature 4 and Patent Literature 5) have been heretofore reported as NaPi-IIb inhibitors. Dihydropyridazine-3,5-dione derivatives typified by 7-[[2,3-difluoro-4-[2-[2-methoxyethyl(methyl)amino]ethoxy]phenyl]methyl]-10-hydroxy-6-methyl-8-oxo-*N*-[4-(trifluoromethyl)-2-[6-(trifluoromethyl)pyrimidin-4-yl]phenyl]-6,7-diazaspiro[4,5]dec-9-ene-9-carboxamide of formula 1 (hereinafter, also referred to as a compound I) have been reported to have inhibitory actions on NaPi-IIb, PiT-1 and PiT-2 (Patent Literature 6 and Patent Literature 7).

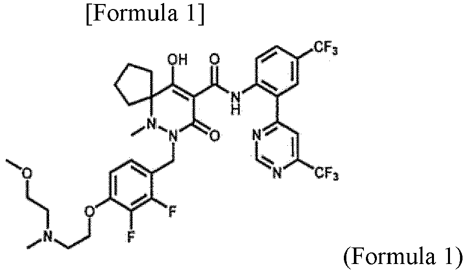

In Patent Literature 6 and Patent Literature 7, a compound obtained by a Suzuki coupling reaction between a phenylboronic acid ester derivative and a chloropyrimidine derivative is used as an intermediate to produce a compound of formula 1. In production of the compound which includes the Suzuki coupling reaction as a reaction step, there is production cost problem. For example, there is a case where palladium used as a catalyst increases the production cost and it is known that bis(pinacolato)diboron used as a raw material for a boronic acid ester increases the raw material cost (Non Patent Literature 9 and Patent Literature 8).

Non Patent Literature 10 discloses a method for producing a salt of a compound of Formula 1 and a crystal thereof. In addition, it is desirable that among crystal polymorphs, a crystal form most thermodynamically stable around room temperature be selected for a drug substance as a solid low-molecular-weight compound, it is known that a most stable form is selected as a crystal of a product to be developed, and establishment of a robust method for production thereof is an important factor directly linked to development cost (Non Patent Literature 11).

### CITATION LIST

### PATENT LITERATURE

[Patent Literature 1] International Publication No. WO 2012/006475
[Patent Literature 2] International Publication No. WO 2011/136269
[Patent Literature 3] International Publication No. WO 2013/062065
[Patent Literature 4] International Publication No. WO 2014/003153
[Patent Literature 5] International Publication No. WO 2018/034883
[Patent Literature 6] International Publication No. WO 2014/142273
[Patent Literature 7] International Publication No. WO 2016/039458
[Patent Literature 8] International Publication No. WO 2019/142854

### NON PATENT LITERATURE

[Non Patent Literature 1] Hruska, KA et al., Kidney Int., 2008, 74(2), 148-157.
[Non Patent Literature 2] "Chapter 1: Definition and classification of CKD" Kidney Int. Suppl., 2013, 3(1), 19-62.
[Non Patent Literature 3] Levey, AS et al., Kidney Int., 2005, 67(6), 2089-2100.
[Non Patent Literature 4] Ritter, CS et al., Clin. J.Am.Soc.Nephrol.2016, 11(6), 1088-1100.
[Non Patent Literature 5] Miyamoto, K et al., J. Pharm. Sci., 2011, 100(9), 3719-3730.
[Non Patent Literature 6] Sabbagh, Y et al., J. Am. Soc. Nephrol., 2009, 20(11), 2348-2358.
[Non Patent Literature 7] Forster IC et al., Mol Aspects Med., 2013, 34(2-3), 386-395.
[Non Patent Literature 8] Lederer E et al., Eur. J. Physiol., 2019, 471(1), 137-148.
[Non Patent Literature 9] Vijayalakshmi C et al., Lett. Org. Chem., 2020, 17(1), 68-72.
[Non Patent Literature 10] Japan Institute for Promoting Invention and Innovation, Journal of technical disclosure, Technique No. 2017-501666
[Non Patent Literature 11] Chemburkar SR et al., Org. Proc. Res. Dev. 2000, 4(5), 413-417.

### SUMMARY OF INVENTION

### TECHNICAL PROBLEM

It is considered that a dihydropyridazine-3,5-dione derivative is used for a therapeutic drug for chronic diseases such as hyperphosphatemia, that is, treatment by administration of the drug is lengthy. For securing stable supply of medicament and reducing the production cost, the inventors of the present application conducted detailed studies on a method for producing the drug substance in terms of raw material cost and reaction conditions.

Specifically, the inventors conducted studies on a method for producing ap-toluenesulfonate of 7-[[2,3-difluoro-4-[2-[2-methoxyethyl(methyl)amino]ethoxy]phenyl]methyl]-10-hydroxy-6-methyl-8-oxo-N [4-(trifluoromethyl)-2-[6-(trifluoromethyl)pyrimidin-4-yl]phenyl]-6,7-diazaspiro[4,5]dec-9-ene-9-carboxamide of formula 1. 2-(4,4,5,5-Tetramethyl-1,3,2-dioxaborolan-2-yl)-4-(trifluoromethyl)aniline used as a raw material for 4-(trifluoromethyl)-2-(6-(trifluoromethyl)pyrimidin-4-yl)aniline (compound II): 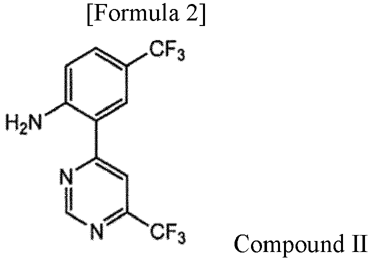 , which is a key intermediate in production of a compound of formula 1, is expensive, significantly affects the original cost for production, and is a cause of the increased production cost for the drug substance. In addition, it was found that in a step of producing a specific crystal form of a *p*-toluenesulfonate of a compound of formula 1, which can be an active ingredient, there arose problems such as slow crystallization and oiling-out in the solution, leading to a decrease in yield of the target substance and contamination by different crystal forms.

### SOLUTION TO PROBLEM

The present inventors have extensively conducted studies for solving these problems. The inventors have found a method for obtaining a *p*-toluenesulfonate of a compound of formula 1 using a raw material as an alternative to a phenylboronic acid ester derivative which is expensive among raw materials, where the method does not involve an increase in the amount of impurities mixed in the drug substance in comparison with conventional methods. Further, it has been found by removing a solvent after obtaining a specific solvate of a *p*-toluenesulfonate of a compound of formula 1 as opposed to a conventional method in which a compound of formula 1 is mixed with *p*-toluenesulfonic acid to obtain a *p*-toluenesulfonate of the compound of formula 1, a target crystal can be acquired with good yield and the target crystal is obtained without ingress of another crystal form. Thus, the present invention has been completed.

Specifically, the disclosure of the following invention is encompassed herein.
[1-1] A method for producing crystalline powder of a *p*-toluenesulfonate of a compound of formula 1: 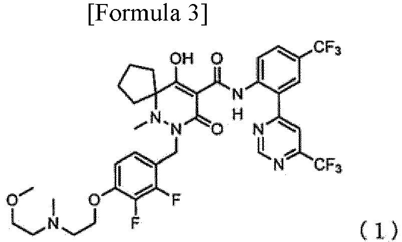 the method comprising:
   converting the compound of formula 1 into the *p*-toluenesulfonate in a solvent and collecting a precipitated solid to obtain wet powder of the *p*-toluenesulfonate; and
   subjecting the wet powder to drying conditions to obtain dry powder of Form I crystal of the *p*-toluenesulfonate.
[1-2] The method according to [1-1], comprising:
   A1) preparing an ethanol solution of a compound of formula 1 and *p*-toluenesulfonic acid;
   A2) adding a poor solvent to the solution to precipitate a solid;
   A3) collecting the solid to obtain wet powder of the *p*-toluenesulfonate; and
   A4) subjecting the wet powder to drying conditions to obtain dry powder of Form I crystal of the *p*-toluenesulfonate.
[1-3] The method according to [1-2], wherein the amount of the poor solvent used in A2 is 0.8 parts by weight or more and 3.5 parts by weight or less based on 1 part by weight of the ethanol used in A1.
[1-4] The method according to [1-1], comprising:
   B1) dissolving a compound of formula 1 in a mixed solvent containing ethanol and a poor solvent to obtain a solution;
   B2) adding an ethanol solution of *p*-toluenesulfonic acid to the solution to precipitate a solid;
   B3) collecting the solid to obtain wet powder of the *p*-toluenesulfonate; and
   B4) subjecting the wet powder to drying conditions to obtain dry powder of Form I crystal of the *p*-toluenesulfonate.
[1-5] The method according to [1-4], wherein the amount of the poor solvent used in B1 is 0.8 weight per weight of the poor solvent based on 1 weight per weight of the ethanol used in B 1.
[1-6] The method according to [1-4], wherein the amount of the poor solvent used in B1 is 0.8 parts by weight or more and 3.5 weight per weight or less based on 1 weight per weight of the ethanol used in B1.
[1-7] The method according to any one of [1-1] to [1-6], comprising further adding a poor solvent to a mixture containing the solid before the collection.
[1-8] The method according to any one of [1-2] to [1-7], wherein the poor solvent is a solvent containing hexane or heptane; a solvent selected from hexane and heptane; or a mixed solvent containing hexane and heptane.
[1-9] The method according to any one of [1-1] to [1-8], wherein the dry powder of the Form I crystal of the *p*-toluenesulfonate has an X-ray powder diffraction pattern including at least one selected from peaks at 4.9°, 9.4°, 9.9°, 15.2°, 15.8°, 18.9° and 22.6° (± 0.5°) as a diffraction angle 2θ in X-ray powder diffraction.
[1-10] The method according to any one of [1-1] to [1-9], wherein the dry powder of the Form I crystal of the *p*-toluenesulfonate has an X-ray powder diffraction pattern including at least one selected from peaks at 4.9°, 9.4°, 15.8°, 18.9° and 22.6° (± 0.5°) as a diffraction angle 2θ in X-ray powder diffraction.
[1-11] The method according to any one of [1-1] to [1-10], wherein the dry powder of the Form I crystal of the *p*-toluenesulfonate has an X-ray powder diffraction pattern including a peak at 15.8 ° (± 0.5 °) as a diffraction angle 2θ in X-ray powder diffraction.
[1-12] The method according to any one of [1-1] to [1-11], wherein the dry powder of the Form I crystal of the *p*-toluenesulfonate has an X-ray powder diffraction pattern including at least one selected from peaks at 4.9°, 9.4°, 9.9°, 15.2°, 15.8°, 18.9° and 22.6° (± 0.2°) as a diffraction angle 2θ in X-ray powder diffraction.
[1-13] The method according to any one of [1-1] to [1-12], wherein the wet powder is a wet crystal.
[1-14] The method according to [1-13], wherein the wet crystal contains ethanol.
[1-15] The method according to any one of [1-2] to [1-14], wherein the poor solvent contains heptane, hexane or pentane.
[1-16] The method according to any one of [1-1] to [1-15], wherein the dry powder of the Form I crystal of the *p*-toluenesulfonate has an X-ray powder diffraction pattern including peaks at 4.9°, 9.4°, 9.9°, 15.2°, 15.8°, 18.9° and 22.6° (± 0.5°) as a diffraction angle 2θ in X-ray powder diffraction.
[1-17] The method according to any one of [1-1] to [1-16], wherein the dry powder of the Form I crystal of the *p*-toluenesulfonate has an X-ray powder diffraction pattern including peaks at 4.9°, 9.4°, 15.8°, 18.9° and 22.6° (± 0.5°) as a diffraction angle 2θ in X-ray powder diffraction.
[1-18] The method according to any one of [1-1] to [1-17], wherein the dry powder of the Form I crystal of the *p*-toluenesulfonate has an X-ray powder diffraction pattern including a peak at 15.8° (± 0.5°) as a diffraction angle 2θ in X-ray powder diffraction.
[1-19] The method according to any one of [1-1] to [1-18], wherein the dry powder of the Form I crystal of the *p*-toluenesulfonate has an X-ray powder diffraction pattern including peaks at 4.9°, 9.4°, 9.9°, 15.2°, 15.8°, 18.9° and 22.6° (± 0.2°) as a diffraction angle 2θ in X-ray powder diffraction.
[1-20] The method according to any one of [1-1] to [1-19], wherein the dry powder of the Form I crystal of the *p*-toluenesulfonate has an X-ray powder diffraction pattern including peaks at 4.9°, 9.4°, 15.8°, 18.9° and 22.6° (± 0.2°) as a diffraction angle 2θ in X-ray powder diffraction.
[1-21] The method according to any one of [1-1] to [1-20], wherein the amount of the solvent used for converting a compound of formula 1 into the *p*-toluenesulfonate is 2 to 15 weight per weight based on 1 weight per weight of the compound of formula 1.
[1-22] The method according to any one of [1-1] to [1-21], wherein the temperature of the solution is in the range of -5°C or higher and 70°C or lower when the solid is precipitated.
[1-23] The method according to any one of [1-1] to [1-22], wherein the amount of *p-*toluenesulfonic acid used for converting a compound of formula 1 into the *p*-toluenesulfonate is in the range of 1 equivalent or more and 1.2 equivalent or less based on 1 equivalent of the compound of formula 1.
[2-1] A method for producing a compound of formula 2: 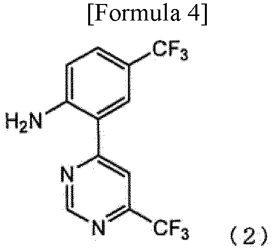 the method comprising:
   treating a compound of formula 3: 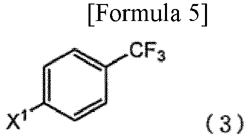
   wherein X¹ is a leaving group,
      with a C₁₋₆ alkylmagnesium halide, a C₁₋₆ alkyllithium and a zinc halide, and reacting the treated compound with a compound of formula 4: 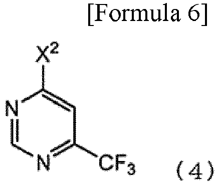
   wherein X² is a leaving group,
      in the presence of a palladium catalyst to obtain a compound of formula 5: 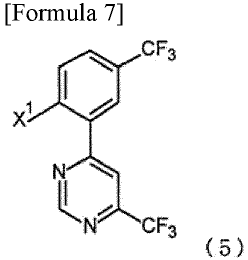 ; and
      reacting the compound of formula 5 with an alkali metal salt of a dicarboxylic acid imide, followed by a decomposition reaction of the resulting imide compound to obtain a compound of formula 2.
[2-2] The method according to [2-1], wherein X¹ is a halogen atom.
[2-3] The method according to [2-1] or [2-2], wherein X¹ is a fluorine atom.
[2-4] The method according to any one of [2-1] to [2-3], wherein X² is a chlorine atom.
[2-5] The method according to any one of [2-1] to [2-4], wherein the C₁₋₆ alkylmagnesium halide is a C₁₋₆ alkylmagnesium chloride.
[2-6] The method according to any one of [2-1] to [2-5], wherein the zinc halide is selected from the group consisting of ZnCl₂, ZnBr₂ and ZnI₂.
[2-7] The method according to any one of [2-1] to [2-6], wherein the palladium catalyst is selected from the group consisting of a 1,1'-bis(diphenylphosphino)ferrocene-palladium(II) dichloride complex, a 1,1'-bis(diphenylphosphino)ferrocene-palladium(II) dichloride dichloromethane complex, bis(triphenylphosphine)palladium(II) dichloride, dichlorobis(tricyclohexylphosphine)palladium(II), [1,3-bis(2,6-diisopropylphenyl)imidazol-2-ylidene] (3-chloropyridyl)palladium(II) dichloride, (1,3-bis(2,6-diisopropylphenyl)imidazolidene) (3-chloropyridyl)palladium(II) dichloride and [1,3-bis(2,6-di-3-pentylphenyl)imidazol-2-ylidene] (3-chloropyridyl)palladium(II) dichloride.
[2-8] The method according to any one of [2-1] to [2-7], wherein the alkali metal salt of a dicarboxylic acid imide is potassium phthalimide.
[2-9] The method according to any one of [2-1] to [2-8], wherein the imide decomposition reaction is carried out by a reaction of the imide compound with a nucleophilic agent selected from hydrazine hydrate and methylamine.
[2-10] The method according to any one of [2-1] to [2-9], wherein the reaction of a compound of formula 5 with an alkali metal salt of a dicarboxylic acid imide is carried out in a solvent selected from the group consisting of *N*,*N*-dimethylformamide, *N,N-*dimethylacetamide, *N*-methylpyrrolidone, 1,3-dimethyl-2-imidazolidinone, *N,N'-*dimethylpropylene urea, acetonitrile and dimethylsulfoxide, or a mixture of two or more selected from the solvents.
[2-11] The method according to any one of [2-1] to [2-10], wherein the reaction for obtaining a compound of formula 5 is carried out in a solvent selected from tetrahydrofuran, 2-methyltetrahydrofuran, dimethoxyethane, 1,4-dioxane and 1,3-dioxane, or a reaction solvent containing two or more selected from the solvents.
[2-12] The method according to any one of [2-1] to [2-11], wherein the reaction for obtaining a compound of formula 5 is carried out in a solvent selected from tetrahydrofuran, 2-methyltetrahydrofuran, and dimethoxyethane, or a reaction solvent containing two or more selected from the solvents.
[2-13] The method according to any one of [2-1] to [2-12], wherein the reaction for obtaining a compound of formula 5 is carried out in a solvent containing tetrahydrofuran or 2-methyltetrahydrofuran, or a reaction solvent containing a mixture of the solvents.
[2-14] The method according to any one of [2-1] to [2-13], wherein a C₁₋₆ alkyllithium is added dropwise to a reaction system in which a compound of formula 3 and a C₁₋₆ alkylmagnesium halide are present, followed by dropwise addition of a zinc halide to the reaction system.
[2-15] The method according to any one of [2-1] to [2-14], wherein a solution of a palladium catalyst and a compound of formula 4 is added dropwise to a reaction system containing a C₁₋₆ alkylmagnesium halide, a C₁₋₆ alkyllithium and a compound of formula 3 treated with a zinc halide.
[3-1] A method for producing a compound of formula 1: 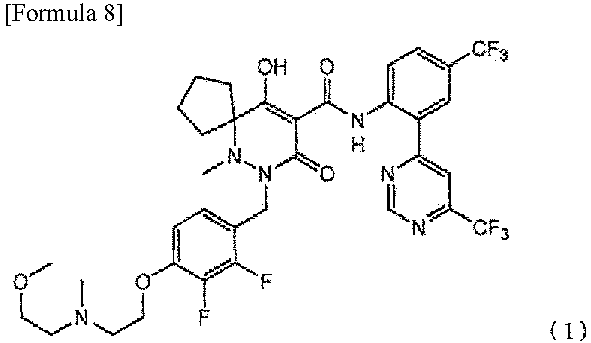 the method comprising:
   1) preparing a compound of formula 2 by the method according to any one of [2-1] to [2-15];
   2) reacting the compound of formula 2 with a malonic acid derivative to prepare a compound of formula 6: 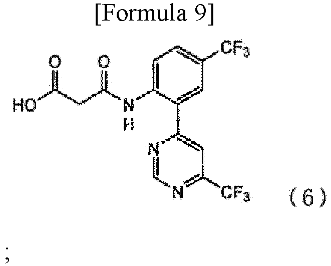
   3) reacting the compound of formula 6 with a compound of formula 7: 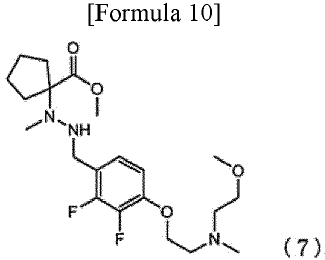 in the presence of a condensing agent to prepare a compound of formula 8: 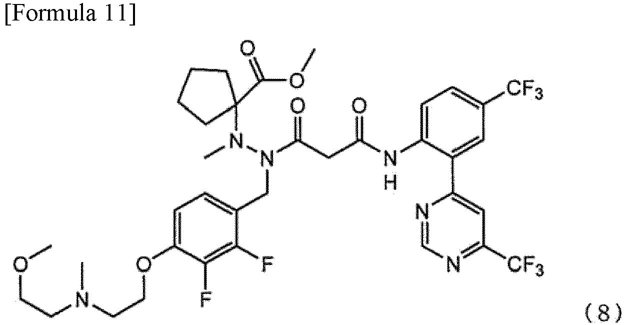 ; and
   4) subjecting the compound of formula 8 to a cyclization reaction to prepare the compound of formula 1.

[3-2] The method according to [3-1], wherein the malonic acid derivative is selected from the group consisting of Meldrum's acid, a dialkylmalonic acid and malonic acid.
[3-3] The method according to [3-1] or [3-2], wherein the reaction of a compound of formula 2 with a malonic acid derivative is carried out in a solvent selected from the group consisting of toluene, heptane, acetonitrile, methanol and ethanol, or a mixture of two or more selected from the solvents.
[3-4] The method according to any one of [3-1] to [3-3], wherein the condensing agent is selected from the group consisting of propylphosphonic anhydride (cyclic trimer), diethyl chlorophosphate and *N*,*N*'-diisopropylcarbodiimide.
[3-5] The method according to any one of [3-1] to [3-4], wherein the reaction of a compound of formula 6 with a compound of formula 7 is carried out in the presence of a base selected from the group consisting of pyridine, *N,N*-diisopropylethylamine and triethylamine in a solvent selected from the group consisting of *N*,*N*-dimethylformamide, *N,N-*dimethylacetamide, *N*-methylpyrrolidone, 1,3-dimethyl-2-imidazolidinone, *N,N'-*dimethylpropylene urea, acetonitrile and dimethylsulfoxide, or a mixture of two or more selected from the solvents.
[3-6] The method according to any one of [3-1] to [3-5], wherein the cyclization reaction of a compound of formula 8 is carried out in the presence of a base selected from the group consisting of potassium carbonate, sodium carbonate, cesium carbonate and potassium phosphate in a solvent selected from the group consisting of methanol, ethanol, 1-propanol, 2-propanol, 1-butanol and acetonitrile.
[4-1] A method for producing a crystal of a *p*-toluenesulfonate of a compound of formula 1: 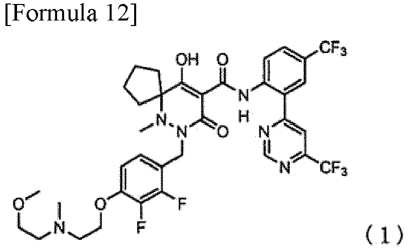 the method comprising:
   converting the compound of formula 1 into the *p*-toluenesulfonate in a solvent and collecting a precipitated solid to obtain the wet *p*-toluenesulfonate; and
   subjecting the wet *p*-toluenesulfonate to drying conditions to obtain a dry Form I crystal of the *p*-toluenesulfonate.
[4-2] The method according to [4-1], wherein the wet *p*-toluenesulfonate is powder.
[4-3] The method according to [4-1] or [4-2], wherein the dry Form I crystal of the p-toluenesulfonate is powder.

### ADVANTAGEOUS EFFECTS OF INVENTION

The present invention provides an efficient method for producing a *p-*toluenesulfonate of 7-[[2,3-difluoro-4-[2-[2-methoxyethyl(methyl)amino]ethoxy]phenyl]methyl]-10-hydroxy-6-methyl-8-oxo-*N-*[4-(trifluoromethyl)-2-[6-(trifluoromethyl)pyrimidin-4-yl]phenyl]-6,7-diazaspiro[4,5]dec-9-ene-9-carboxamide (compound I) which has a strong inhibitory action on NaPi-IIb, PiT-1 and PiT-2.

### BRIEF DESCRIPTION OF DRAWINGS

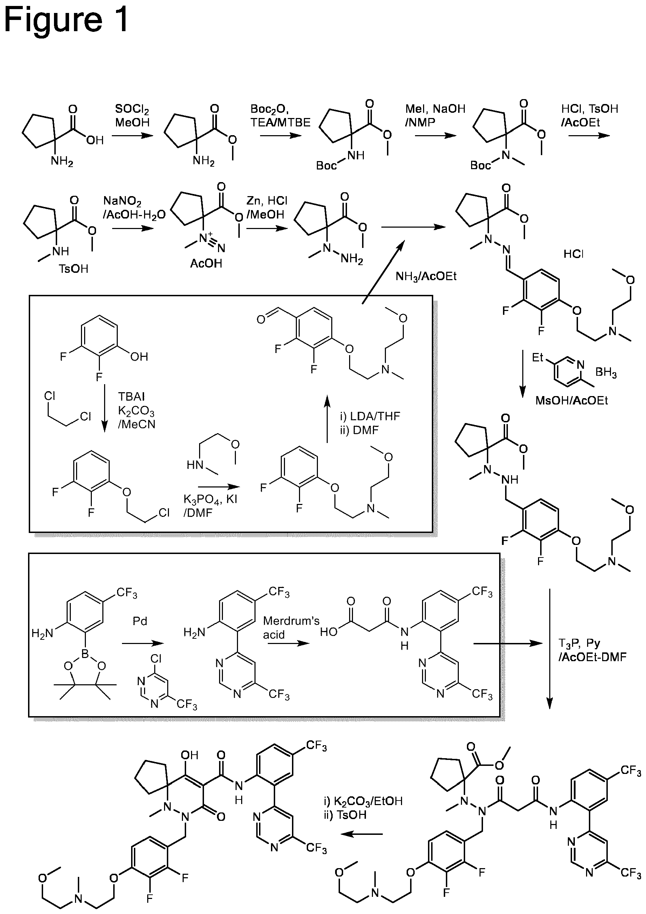

Figure 1 shows an example of a synthesis scheme for a compound I.
Figure 2 shows the results of X-ray powder diffraction measurement of crystal powder obtained in Example 7, and the X-ray powder diffraction patterns of crystals obtained by other methods. The ordinate represents a diffraction intensity, and the abscissa represents a diffraction angle 2θ (°).
Figure 3 shows the result of thermogravimetric analysis of dry powder of Example 7. The abscissa represents a temperature (°C), and the ordinate represents a heat flow observed in thermal analysis.
Figure 4 shows the results of X-ray powder diffraction measurement of crystal powder obtained in Example 8, and the X-ray powder diffraction patterns of crystals obtained by other methods. The ordinate represents a diffraction intensity, and the abscissa represents a diffraction angle 2θ (°).
Figure 5 shows the result of thermogravimetric analysis of dry powder of Example 8. The abscissa represents a temperature (°C), and the ordinate represents a heat flow observed in thermal analysis.

### DESCRIPTION OF EMBODIMENTS

The abbreviations as used herein are listed below.
DIC: *N*,*N*'-diisopropylcarbodiimide
DIPEA: *N*,*N*'-diisopropylethylamine
DMA: *N*,*N*'-dimethylacetamide
DMF: *N*,*N*'-dimethylformamide
DMI: 1,3-dimethyl-2-imidazolidinone
DMSO: dimethylsulfoxide
DMPU: *N*,*N*'-dimethylpropylene urea
EtOH: ethanol
GC: gas chromatography
HPLC: high-performance liquid chromatography
MeCN: acetonitrile
MeOH: methanol
MTBE: methyl *tert*-butyl ether
NMP: *N*-methylpyrrolidone
NMR: nuclear magnetic resonance spectrum
PDA: photodiode array detector
T3P: propylphosphonic anhydride
*t*-Bu: *t*-butyl
TEA: triethylamine
TFA: trifluoroacetic acid

### (Definition of functional group)

As used herein, the term "leaving group" refers to a group which can be desorbed by cleavage of a chemical bond, and is capable of generating an anionic atom, an anionic molecule or the like, halogeno groups containing a halogen atom, such as a fluoro group, a chloro group, a bromo group and an iodo group, and sulfonyl groups such as a mesyl group, a tosyl group, a trifluoromethanesulfonyl group and a nitrophenylsulfonyl group are exemplified.

As the "halogen atom" herein, a fluorine atom, a chlorine atom, a bromo atom and an iodine atom are exemplified.

As used herein, the term "alkyl" is a monovalent group derived from an aliphatic hydrocarbon by removing any one hydrogen atom, and a subset of hydrocarbyl or hydrocarbon group structures which do not contain a hetero atom (which is an atom other than carbon and hydrogen atoms) or an unsaturated carbon-carbon bond and contain hydrogen and carbon atoms in the backbone. The alkyl group include linear, branched or cyclic alkyl groups. The alkyl group is an alkyl group having 1 to 20 carbon atoms (C₁₋₂₀, hereinafter, "C_{p-q}" means that the number of carbon atoms is p to q), and C₁₋₆ alkyl groups are preferable. Specific examples thereof include methyl, ethyl, propyl, butyl, pentyl, hexyl, isopropyl, *tert*-butyl, *sec*-butyl, cyclopentyl and cyclohexyl.

As used herein, the temperature at which the compound is handled, a temperature around an inside temperature of a typical working environment for operators (room temperature), e.g. a temperature in the range of 10°C or higher and 30°C or lower, or in the range of 15°C or higher and 25°C or lower is exemplified. As the temperature at which the compound is handled in a reaction vessel, a temperature range from -100°C associated with use of a cooling medium such as liquid nitrogen to a temperature around the boiling point of the solvent is exemplified. A temperature suitable for efficiently producing a target product can be selected according to the stability of the compound and the reactivity of a starting substance.

In an aspect of the present invention, it is possible to produce 4-(trifluoromethyl)-2-(6-(trifluoromethyl)pyrimidin-4-yl)aniline (compound II) in accordance with a reaction scheme (scheme 1) shown below.

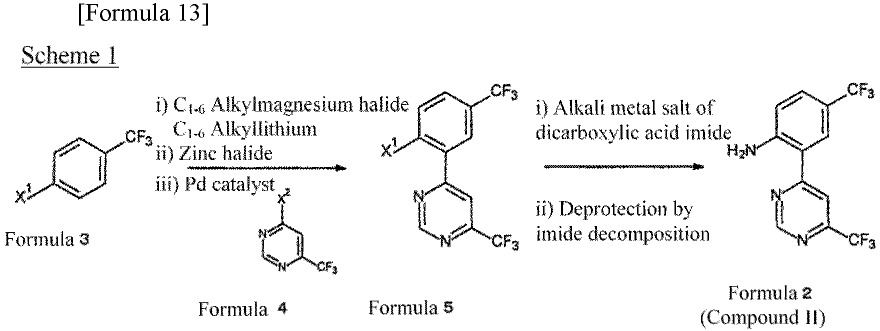

In the formula, X¹ and X² each represent a leaving group. As the compound of formula 3 which is a starting substance, a compound available through purchase can be used. Examples of X¹ as a leaving group include halogen atoms, preferably a fluorine atom or a chlorine atom, particularly preferably a fluorine atom. Examples of X² as a leaving group include halogen atoms, preferably a chlorine atom, a bromine atom or an iodine atom, particularly preferably a chlorine atom.

In an aspect, a C₁₋₆ alkylmagnesium halide and a C₁₋₆ alkyllithium are added to a solution of a compound of formula 3 at, for example, -100 to 10°C, preferably -60 to 0°C, more preferably -30 to -10°C. The molar ratio of the reagents used [C₁₋₆ alkyllithium/C₁₋₆ alkylmagnesium halide] can be, for example, 1.5 to 3.0, preferably 1.8 to 2.8, more preferably 2.0 to 2.5, and it is possible to add the C₁₋₆ alkylmagnesium halide solution and the C₁₋₆ alkyllithium solution in this order. In this case, the amount of the alkylmagnesium halide with respect to a compound of formula 4 is not particularly limited, and can be, for example, 0.25 to 2.0 equivalents, preferably 0.28 to 1.0 equivalents, more preferably 0.30 to 0.50 equivalents. A compound of formula 3 can be used in an amount of, for example, 0.8 to 3.0 equivalents, preferably 1.0 to 2.0 equivalents, more preferably 1.3 to 1.7 equivalents, with respect to a compound of formula 4. The dropwise addition time can be appropriately set with consideration given to a reaction scale and a change in temperature of the reaction solution. A reaction mixture obtained after the dropwise addition of the C₁₋₆ alkyllithium solution can be stirred at, for example, -100 to 10°C, preferably -60 to 0°C, more preferably - 30 to -10°C. The stirring time can be, for example, 0.1 to 12 hours, preferably 1 to 10 hours, more preferably 2 to 7 hours.

Examples of the solvent used for the solution of a compound of formula 3 include ethers (e.g. tetrahydrofuran (THF), 2-methyltetrahydrofuran, diethyl ether, t-butyl methyl ether, diisopropyl ether, cyclopentyl methyl ether and 1,2-dimethoxyethane), and hydrocarbons (e.g. pentane, hexane, heptane, benzene and toluene). Examples of the solvent for the C₁₋₆ alkylmagnesium halide solution include ethers (e.g. tetrahydrofuran (THF), 2-methyltetrahydrofuran, diethyl ether, *t*-butyl methyl ether, diisopropyl ether, cyclopentyl methyl ether and 1,2-dimethoxyethane), and hydrocarbons (e.g. pentane, hexane, heptane, benzene and toluene). Examples of the solvent for the C₁₋₆ alkyllithium solution include hydrocarbons (e.g. pentane, hexane, heptane, benzene and toluene), and ethers (e.g. tetrahydrofuran (THF), 2-methyltetrahydrofuran, diethyl ether, t-butyl methyl ether, diisopropyl ether, cyclopentyl methyl ether and 1,2-dimethoxyethane).

Subsequently, a zinc halide solution is added dropwise to the resulting reaction mixture at, for example, -60 to 10°C, preferably -50 to 0°C, more preferably -30 to -10°C. Here, the zinc halide can be used in an amount of, for example, 0.1 to 2.0 equivalents, preferably 0.6 to 1.5 equivalents, more preferably 0.8 to 1.3 equivalents, with respect to a compound of formula 4. The dropwise addition time can be appropriately set with consideration given to a reaction scale and a change in temperature of the reaction solution. Examples of the solvent used for the zinc halide solution include ethers (e.g. tetrahydrofuran (THF), 2-methyltetrahydrofuran, diethyl ether, t-butyl methyl ether, diisopropyl ether, cyclopentyl methyl ether and 1,2-dimethoxyethane), and hydrocarbons (e.g. pentane, hexane, heptane, benzene and toluene).

A reaction mixture obtained after the dropwise addition of the zinc halide solution can be stirred at, for example, -60 to 10°C, preferably -40 to 10°C, more preferably -20 to 5°C. The stirring time can be, for example, 0.1 to 4 hours, preferably 0.15 to 2 hours, more preferably 0.2 to 1.5 hours. Here, a compound of formula 4 and a palladium catalyst can be added to the reaction mixture at, for example, -20 to 20°C, preferably -20 to 10°C, more preferably -10 to 5°C. Here, the palladium catalyst can be used in an amount of, for example, 0.001 to 0.1 equivalents (0.1 to 10 mol%), preferably 0.003 to 0.05 equivalents (0.3 to 5 mol%), more preferably 0.005 to 0.02 equivalents (0.5 to 2 mol%), with respect to a compound of formula 4.

The reaction mixture obtained by adding a compound of formula 4 and the palladium catalyst is heated at, for example, 25 to 100°C, preferably 40 to 80°C, more preferably 50 to 70°C, and here, stirring can be carried out for, for example, 0.1 to 12 hours, preferably 0.5 to 8 hours, more preferably 1 to 3 hours.

Post-treatment of the reaction mixture after addition of a compound of formula 4 and the palladium catalyst can be performed after the decreased amount or disappearance of the compound of formula 4 in the reaction system is confirmed by analysis by HPLC, GC or the like. The resulting compound of formula 5 may be subjected to a next step after being purified by column chromatography or the like, or may be subjected to a next step without being particularly purified.

In an aspect of the step, examples of the C₁₋₆ alkylmagnesium halide include C₁₋₆ alkylmagnesium chlorides, specifically C₃₋₆ alkylmagnesium chlorides, and i-propylmagnesium chloride, *n*-butylmagnesium chloride, cyclopentylmagnesium chloride and cyclohexylmagnesium chloride are preferable. These C₁₋₆ alkylmagnesium halides can be used as a solution in an appropriate solvent.

In an aspect of the step, the C₁₋₆ alkyllithium is not particularly limited, and examples thereof include C₁₋₆ alkyllithiums easily available as an industrial raw material, specifically methyllithium, *n*-butyllithium, s-butyllithium and *t*-butyllithium. The C₁₋₆ alkyllithium can be used as a solution in an appropriate solvent.

In an aspect of the step, the zinc halide is not particularly limited, and examples thereof include zinc halides easily available as industrial raw materials, specifically zinc chloride, zinc bromide or zinc iodide. These zinc halides can be used as a solution in an appropriate solvent.

In an aspect of the step, the palladium catalyst is not particularly limited as long as it can act as a catalyst of the reaction, but examples of the palladium catalyst include 1,1'-bis(diphenylphosphino)ferrocene-palladium(II) dichloride complexes, 1,1'-bis(diphenylphosphino)ferrocene-palladium(II) dichloride dichloromethane complexes, bis(triphenylphosphine)palladium(II) dichloride, dichlorobis(tricyclohexylphosphine)palladium(II), [1,3-bis(2,6-diisopropylphenyl)imidazol-2-ylidene] (3-chloropyridyl)palladium(II) dichloride, (1,3-bis(2,6-diisopropylphenyl)imidazolidene) (3-chloropyridyl)palladium(II) dichloride and [1,3-bis(2,6-di-3-pentylphenyl)imidazol-2-ylidene] (3-chloropyridyl)palladium(II) dichloride.

In an aspect of the step, X¹ is a fluorine atom, X² is a chlorine atom, the C₁₋₆ alkylmagnesium halide is a C₃₋₆ alkylmagnesium chloride, and the zinc halide is zinc chloride. In another aspect of the step, X¹ is a fluorine atom, X² is a chlorine atom, the C₁₋₆ alkylmagnesium halide is a C₃₋₆ alkylmagnesium chloride, the zinc halide is zinc chloride, and the palladium catalyst is a 1,1'-bis(diphenylphosphino)ferrocene-palladium(II) dichloride complex or a 1,1'-bis(diphenylphosphino)ferrocene-palladium(II) dichloride dichloromethane complex.

In an aspect, a compound of formula 5 can be reacted with an alkali metal salt of a dicarboxylic acid imide in an appropriate solvent at an appropriate temperature, for example, 50 to 150°C, preferably 80 to 130°C, more preferably 90 to 110°C. Here, the alkali metal salt of a dicarboxylic acid imide alkali metal salt can be used in an amount of, for example, 0.9 to 2.0 equivalents, preferably 1.0 to 1.5 equivalents, more preferably 1.05 to 1.2 equivalents, with respect to a compound of formula 5. The reaction time can be appropriately set with consideration given to a reaction scale and a change in temperature of the reaction solution. As the solvent, for example, acetonitrile, dimethylsulfoxide (DMSO), *N*,*N*-dimethylformamide (DMF), *N*,*N*-dimethylacetamide (DMA), *N*-methylpyrrolidone (NMP), 1,3-dimethyl-2-imidazolidinone (DMI), *N*,*N*'-dimethylpropylene urea (DMPU), or a mixture of two or more selected from the solvents can be used. Preferably, dimethylsulfoxide, *N*,*N*-dimethylformamide, *N*,*N*-dimethylacetamide, *N*-methylpyrrolidone or the like can be used. As the alkali metal salt of a dicarboxylic acid imide, potassium phthalimide or sodium phthalimide is exemplified, and potassium phthalimide is preferable.

The reaction mixture is analyzed by HPLC, GC or the like to confirm the decreased amount or disappearance of a compound of formula 5 in the reaction solution, and a nucleophilic agent is then added to the reaction mixture to convert the imide structure part into an amine (imide decomposition reaction). The reaction analysis of the imide decomposition reaction can be omitted. The reaction temperature is not particularly limited, and can be, for example, 0 to 150°C, preferably 25 to 100°C, more preferably 40 to 60°C. Here, as the nucleophilic agent, amines such as C₁₋₆ alkylamines and hydrazine can be used, and the nucleophilic agent is preferably a C₁₋₆ alkylamine, more preferably a methylamine aqueous solution. The amount of the methylamine aqueous solution is not particularly limited as long as a significant side reaction does not occur, and the methylamine aqueous solution can be used in an amount such that the content of methylamine is 2 to 30 equivalents, preferably 3 to 20 equivalents, more preferably 5 to 15 equivalents, in terms of equivalents with respect to a compound of formula 5. The reaction time can be appropriately set with consideration given to a reaction scale and a change in temperature of the reaction solution. A crude product from a compound of formula 2 (compound II) can be purified. Examples of the purification method include column chromatography and recrystallization.

The compound II obtained in the above-described method can be used as a synthesis intermediate in method for producing the compound I as described in a known literature (Patent Literatures 6 and 7 and Non Patent Literature 10).

As an example of the synthesis intermediate for the compound I, more specifically, a compound of formula 6 which is obtained by reacting the compound II with a malonic acid derivative in an appropriate solvent is exemplified.

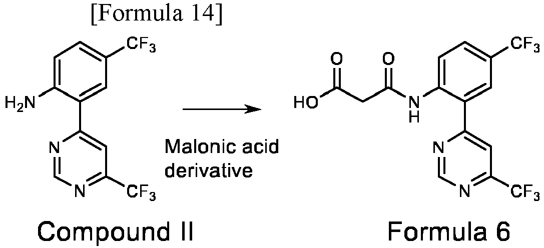

As an aspect of the malonic acid derivative used in the method, Meldrum's acid, a dialkylmalonic acid such as dimethylmalonic acid or diethylmalonic acid, or malonic acid is exemplified. Meldrum's acid is preferable. The solvent used in carrying out this step is not particularly limited and may be a solvent used in an ordinary chemical reaction as long as the relevant reaction is not hindered, and it is preferable to carry out the reaction in a solvent selected from toluene, heptane, acetonitrile, methanol and ethanol, or a mixture of two or more selected from the solvents.

As an example of the synthesis intermediate for the compound I, more specifically, a compound of formula 8 which is obtained by condensing a compound of formula 7 that can be produced by a known method (Patent Literatures 6 and 7) and a compound of formula 6 in an appropriate solvent is exemplified.

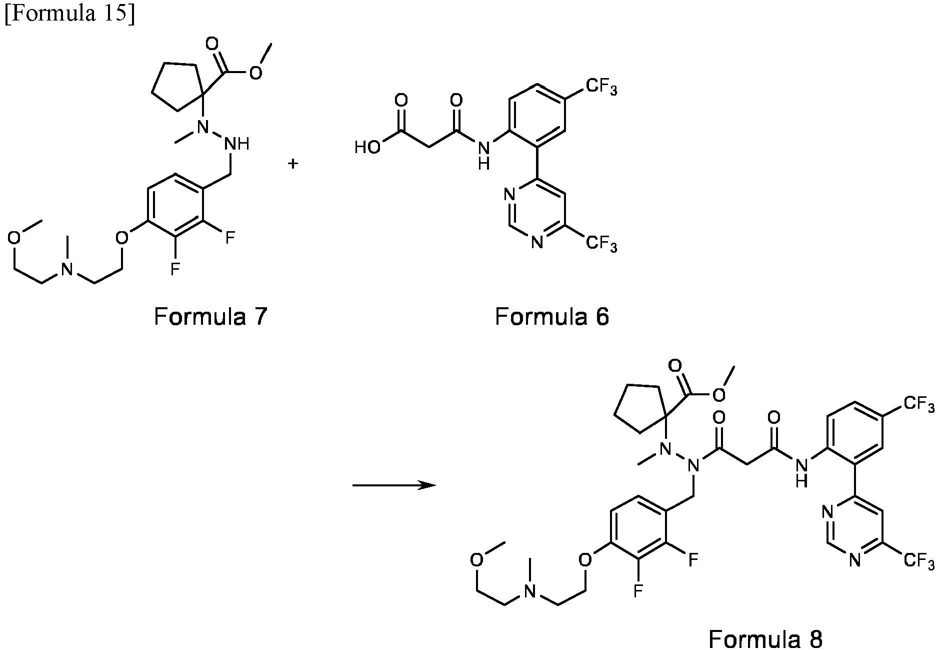

The condensation reaction of a compound of formula 6 with a compound of formula 7 can be carried out by a method which is commonly used for a condensation reaction of a carboxyl group with an amino group by a person skilled in the art. As an aspect of the condensation reaction, mention is made of a condensation reaction using a condensing agent. As the condensing agent, diethyl chlorophosphate, propylphosphonic anhydride (T3P), *N,N'-*dicyclohexylcarbodiimide (DCC), *N*,*N*'-diisopropylcarbodiimide (DIC), 1-(3-dimethylaminopropyl)-3-ethylcarbodiimide (EDCI) or the like is exemplified. Diethyl chlorophosphate or propylphosphonic anhydride (T3P) is preferable. The solvent used in carrying out this step is not particularly limited and may be a solvent used in an ordinary chemical reaction as long as the relevant reaction is not hindered, and it is preferable to carry out the reaction in a solvent selected from *N,N*-dimethylformamide, *N,N*-dimethylacetamide, *N*-methylpyrrolidone, 1,3-dimethyl-2-imidazolidinone, *N*,*N*'-dimethylpropylene urea, acetonitrile and dimethylsulfoxide, or a mixture of two or more selected from the solvents. In addition, the condensation reaction is carried out in the presence of a base if necessary. Specific examples of the base include a base selected from the group consisting of pyridine, dimethylaminopyridine, *N,N*-diisopropylethylamine and triethylamine.

In an aspect of the present invention, a method for producing crystal powder of a *p-*toluenesulfonate (TsOH salt) of the compound I is provided. The outline of the production method is given by a scheme below.

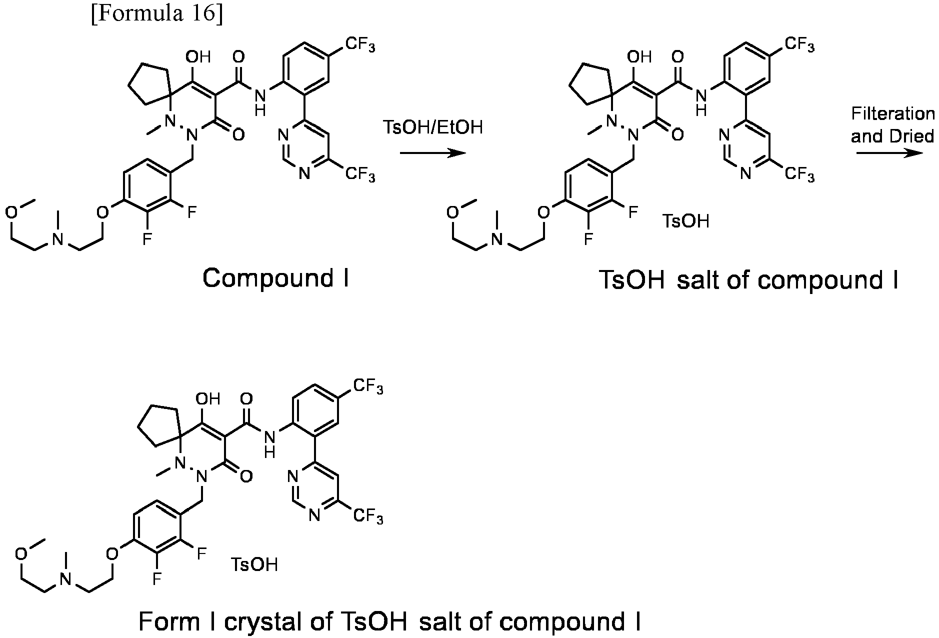

In an aspect of the present invention, a *p*-toluenesulfonate of the compound I can be obtained by precipitating the *p*-toluenesulfonate of the compound I from a solution obtained by dissolving the compound I and *p*-toluenesulfonic acid in a solvent. The amount of *p-*toluenesulfonic acid used can be, for example, 1 equivalent or more, specifically 1 equivalent, with respect to the compound I. A commercialized product can be used as the *p-*toluenesulfonic acid, and *p*-toluenesulfonate monohydrate is preferable.

As the solvent for the solution of the compound I and *p*-toluenesulfonic acid, ethanol can be used. For precipitation of a target *p*-toluenesulfonate, a seed crystal may be added, and/or another solvent may be added in an ethanol solution. As the other solvent, a solvent in which a target product is less soluble (poor solvent) can be used for the purpose of increasing the amount of the target product precipitated, or enhancing the precipitation rate of the target product. Such a poor solvent is hexane, heptane, pentane or the like. As a mixing ratio between ethanol and the other solvent in precipitation of a crystal from the solution, the ratio of parts by weight of the other solvent to 1.0 part by weight of ethanol can be 0.80 to 3.5, preferably 1.0 to 3.0, more preferably 1.2 to 2.5, most preferably 1.5 to 2.2. As the seed crystal, for example, a crystal described in Non Patent Literature 10 can be used.

The "Form I crystal" in the present invention is an aspect of the crystal form of the p-toluenesulfonate of the compound I, and it is indicated that the Form I crystal has peaks at diffraction angles (2θ) around 4.9°, 9.4°, 9.9°, 15.2°, 15.8°, 18.9° and 22.6° in an X-ray powder diffraction pattern (Non Patent Literature 9). The Form I crystal may have an X-ray powder diffraction pattern including at least one selected from the group consisting of peaks at 4.9°, 9.4°, 9.9°, 15.2°, 15.8°, 18.9° and 22.6° (± 0.2°) as a diffraction angle (2θ), or may have an X-ray powder diffraction pattern including all the peaks.

In addition, it is indicated that in thermogravimetric analysis, peaks associated with melting appear at 112.6°C (extrapolation point) and 126.6°C (peak top).

After precipitation of a target *p*-toluenesulfonate, still another solvent may be added for further increasing the amount of precipitation. The other solvent is hexane, heptane, pentane or the like. As a mixing ratio of ethanol and the other solvent after the other solvent is added again, the ratio of the other solvent to 8.0 weight per weight of ethanol can be 7.0 to 28 weight per weight, preferably 8.0 to 24 weight per weight, more preferably 10 to 20 weight per weight, most preferably 14 to 20 weight per weight.

In the above-described step, the ethanol solution can be held at, for example, 22 to 28°C, specifically 25°C for dissolving the compound I and *p*-toluenesulfonic acid. For further increasing the amount of precipitation, the temperature of the crystallization in which the other solvent is added may be set lower than the temperature of the solution in which the compound I and *p*-toluenesulfonic acid are dissolved. The temperature can be, for example, -5 to 33°C, specifically 25°C. The temperature of the crystallization in which the other solvent is added can be, for example, -5 to 33°C, specifically 25°C.

In another aspect of the present invention, a *p*-toluenesulfonate of the compound I can be obtained by dissolving the compound I in a mixture of ethanol and the other solvent, adding an ethanol solution of *p*-toluenesulfonic acid, and then precipitating the *p-*toluenesulfonate of the compound I. The amount of *p*-toluenesulfonic acid used can be, preferably in the range of 1 equivalent or more and 1.2 equivalent or less, more preferably in the range of 1 equivalent or more and 1.2 equivalents or less, most preferably 1 equivalent, with respect to the compound I. Seed crystals may be added for precipitation of a target *p-*toluenesulfonate. The other solvent is hexane, heptane, pentane or the like, preferably heptane, more preferably *n*-heptane. As a mixing ratio between ethanol and the other solvent, the ratio ofthe other solvent to 8.0 weight per weight of ethanol can be 7.0 to 28 weight per weight, preferably 8.0 to 24 weight per weight, more preferably 10 to 20 weight per weight, most preferably 12 to 17 weight per weight. As the seed crystal, for example, a crystal described in Non Patent Literature 10 can be used. When the seed crystal is added to the reaction mixture, dry seed crystals or seed suspension where seed crystals dispersed in an appropriate solvent may be used. When a seed suspension is used, the solvent for dispersion may be a solvent in which the seed crystals are fully dissolved, and is preferably a solvent containing a poor solvent used for precipitating a *p*-toluenesulfonate of the compound I, more preferably a solvent mixture containing *n*-heptane, still more preferably *n*-heptane.

After precipitation of a target *p*-toluenesulfonate of the compound I by addition of the ethanol solution of *p*-toluenesulfonic acid, still another solvent may be added for further increasing the amount of precipitation. The other solvent is solvent which can precipitate the p-toluenesulfonateof the compound I, preferably hexane, heptane, pentane or the like, more preferably solvent containing n-heptane, and further preferably n-heptane. As a mixing ratio of ethanol and the other solvent after the other solvent is added again, the ratio of the other solvent to 8.0 weight per weight of ethanol can be 7.0 to 28 weight per weight, preferably 8.0 to 24 weight per weight, more preferably 10 to 20 weight per weight, most preferably 12 to 17 weight per weight.

In the above-described step, the ethanol solution can be held at, for example, 22 to 28°C, specifically 25°C for dissolving the compound I. The temperature of the crystallization in which the other solvent is added can be, for example, -5 to 33°C, specifically 25°C. The temperature of the crystallization in which the other solvent is further added can be, for example, -5 to 33°C, specifically 25°C.

The precipitated crystal can be isolated and purified from the dissolving solution or mixed solution by known separation and purification means such as solid-liquid separation operation, for example, separation of solid components from liquid components by filtration or centrifugation, washing of solid components with a washing solvent, and reduced-pressure drying of the washing solvent or the like attached on the solid components. The washing solvent for solid components is preferably solvent in which the desired crystal of the *p-*toluenesulfonate of compound I is not transferred to another crystal, solvent in which the solubility of the *p*-toluenesulfonate of compound I is low, and/or solvent in which the *p-*toluenesulfonate of compound I is not decomposed, for example hexane, heptane, isopropyl acetate, *n*-butyl acetate and *t*-butyl methyl ether, or a mixture of these solvents.

As used herein, the term "wet" refers to a state in which a solid contains a solvent. For example, when a solid collected by solid-liquid separation operation contains a part of the separated liquid, the solid can be referred to as a wet solid. In an aspect of the present invention, the *p*-toluenesulfonate of the compound I precipitated in the solvent is collected as a wetted solid (i.e. wet solid), and the wet solid may be wet powder and/or a wet solid.

As used herein, the term "powder" means a solid having a particle size of 0.5 to 20 µm, for example, 1.0 to 10 µm. The particle size distribution of the powder can be measured by information from, for example, a laser diffraction/scattering method.

As used herein, the term "wet powder" means a mixture of powder collected by solid-liquid separation operation and a solvent used for the solid-liquid separation operation and/or a solvent used for washing the powder. The mixing ratio between the powder and the solvent may be arbitrary.

As used herein, the term "wet crystal" means a mixture of a crystal precipitated by crystallization operation and a solvent used for the crystallization operation and/or a solvent used for washing the crystal. The mixing ratio between the crystal and the solvent may be arbitrary.

As used herein, the term "dry powder" refers to powder in which in contrast to the wet powder, solvent components are vaporized, so that the amount of solvent components in the powder decreases. The residual amount of the solvent with respect to the powder may be arbitrary, and the dry powder refers to powder in which the amount of the solvent in the powder decreases, so that adhesion between powders is reduced to improve fluidity.

As used herein, the term "drying conditions" is not particularly limited, and refers to, for example, conditions for converting a wet solid (e.g. wet powder) into a dry solid (e.g. dry powder). When left standing at normal temperature and pressure, wet powder contains a solvent which is easily vaporized at normal temperature can be converted into dry powder as the solvent is evaporated. The wet powder can be heated for accelerating vaporization of the solvent. When heating is not suitable due to the nature of substances forming the powder, the wet powder may be placed under reduced pressure to accelerate vaporization of the solvent. Heating and pressure reduction may be performed simultaneously or separately.

In an aspect of the present invention, by subjecting a wet powder of a *p-*toluenesulfonate of the compound I to drying conditions, a dry powder of Form I crystal of the *p*-toluenesulfonate of the compound I can be obtained. In an aspect, by phase transition of the crystal of the p-toluenesulfonate of the compound I contained in the wet powder under drying conditions, a dry powder of Form I crystal of a *p*-toluenesulfonate of the compound I is obtained.

As used herein, the term "X-ray powder diffraction" refers to a numerical value which can be determined using an X-ray diffraction phenomenon that is used for identification and structural analysis of a crystalline substance, and the value is specific to certain powder. This value is typically represented by one or more diffraction angles (2θ value). The X-ray powder diffraction can be measured with a CuKα1 radiation, and a person skilled in the art can perform the measurement by using marketed equipment for X-ray powder diffraction measurement in accordance with an instruction manual for the equipment. More specifically, a sample to be measured is irradiated with an X-ray of CuKα1, and a diffraction X-ray is measured with respect to an incident X-ray. In this way, the 2θ value can be measured. For example, the measurement can be performed in accordance with a conventional method such as "X-ray powder Diffractometry" described in Japanese Pharmacopoeia (the 17th edition or the 18th edition).

The diffraction angle (2θ value) in an X-ray powder diffraction spectrum may have some margin of error depending on measurement equipment or measurement conditions such as diffraction angle reading conditions. Herein, the diffraction angle may have a margin of error in measurement within about ± 0.5° to ± 0.2°.

The term "(± 0.2°)" described at the end of listed diffraction angles 2θ in the written diffraction angles 2θ herein means that the range of ± 0.2° with respect to each described value is acceptable for all the listed diffraction angles 2θ. The term "(± 0.5°)" described at the end of listed diffraction angles 2θ means that the range of ± 0.5° with respect to each described value is acceptable for all the listed diffraction angles 2θ.

As used herein, the term "X-ray powder diffraction pattern" means a pattern obtained by plotting a diffraction angle from diffraction, which is obtained by measurement of an X-ray powder diffraction spectrum, and the intensity thereof on the abscissa and the ordinate, respectively. A person skilled in the art can perform the plotting by using marketed equipment for X-ray powder diffraction measurement in accordance with an instruction manual for the equipment.

As used herein, the term "thermogravimetric analysis" refers to a method for thermally analyzing a change in physical and chemical properties of a sample, which is analysis means for measuring a weight change caused by heating a sample. A person skilled in the art can perform the measurement by using marketed equipment for thermogravimetric analysis measurement in accordance with an instruction manual for the equipment.

As used herein, the term "differential thermal analysis" refers to analysis means for detecting and measuring heat generation or heat absorption caused by heating a sample. A person skilled in the art can perform the measurement by using marketed equipment for differential thermal analysis measurement in accordance with an instruction manual for the equipment. Use of "thermogravimetric analysis" and/or "differential thermal analysis" enables acquisition of information on a physical phenomenon such as sublimation, melting, solidification, condensation, evaporation, decomposition, adsorption or desorption of the sample.

In thermogravimetric analysis, an endothermic peak (peak top value) measured may have a change in measured temperature depending on the width of a temperature increase per minute, the impurity of a sample, or the like. Herein, the measured values of the extrapolation point and the peak top may have a margin of error in measurement within about ± 5.0°C.

Crystal powder obtained by the method of the present invention is ground or is not ground, and can be processed into pharmaceutical compositions in various forms, for example, oral preparations such as tablets, capsules, granules, subtle granules, powders and dry syrups, or injection preparations, and it is preferable to use the crystal powder for oral preparations. These pharmaceutical compositions can be produced by a conventional formulation method known to a person skilled in the art with the use of a pharmaceutically acceptable carrier. When an oral solid preparation is prepared, an excipient, and a binder, a disintegrant, a lubricant, a colorant, a taste improving agent, an odor improving agent and the like if necessary can be added to an active ingredient, followed by production of a tablet, a coated tablet, a granule, a powder, a dry syrup, a capsule or the like by a conventional method. When an oral liquid preparation is prepared, a taste improving agent, a buffering agent, a stabilizer, an odor improving agent and the like can be added to an active ingredient, followed by production of an oral solution, a syrup or the like by a conventional method. When an injection preparation is prepared, a pH adjuster, a buffering agent, a stabilizer, a tonicity agent, a regional anesthetic and the like can be added to an active ingredient, followed by production of a subcutaneous, intramuscular or intravenous injection preparation by a conventional method.

The amount of a *p*-toluenesulfonate of the compound I to be added in the pharmaceutical composition varies depending on the symptom of a patient to which the *p-*toluenesulfonate of the compound I is applied, the dosage form thereof, or the like, and in general, it is desirable that the amount of the *p*-toluenesulfonate of the compound I per dosage unit form be about 10 to 700 mg for an oral preparation and about 10 to 700 mg for an injection preparation. In addition, the daily dosage of the Form I crystal in the pharmaceutical composition depends on the symptom, the administration route, the age of a patient, or the like, and cannot be definitely determined. The daily dosage is determined on the basis of a doctor's prescription, and is normally preferably about 10 to 500 mg.

Herein, the meaning of the term "and/or" includes any combination in which "and" and "or" are appropriately combined. Specifically, for example, the term "A, B and/or C" includes the following seven variations; (i) A, (ii) B, (iii) C, (iv) A and B, (v) A and C, (vi) B and C, and (vii) A, B and C.

### EXAMPLES

Hereinafter, the present invention will be described in more detail on the basis of the examples, but the present invention is not limited to these examples.

As a solvent used for carrying out the present invention and exemplified by DMF, DMA, NMP, DMI, DMPU or the like, a product from a commercial supplier was used without being purified. In addition, in a reaction without addition of water as a solvent, a product from a commercial supplier, such as a dehydrated solvent, an super-dehydrated solvent or an anhydrous solvent, was used without being purified.

As reagents used for carrying out the present invention, products from commercial suppliers were used without being purified unless otherwise specified.

The ¹H-NMR spectrum was measured using a nuclear magnetic resonator JNM-ECP 500 (manufactured by JEOL Ltd.), the chemical shift of Me₄Si used as an internal standard substance was set to 0 ppm, and a deuterium lock signal from a sample solvent was consulted. The chemical shift of a signal from a compound to be analyzed was expressed in ppm. Abbreviations for splitting of a signal were expressed as follows: s = singlet, brs = broad singlet, d = doublet, t = triplet, q = quartet, dd = double doublet and m = multiplet. The splitting width of a signal was expressed as a J value (Hz). The integral value of signals was calculated on the basis of a ratio of the signal area intensity of each signal.

The X-ray powder diffraction (XRPD) measurement was carried out under the following conditions, and the 2θ value in the scanning range was calculated. An X-ray diffraction pattern was obtained by plotting a diffraction angle (2θ value) on the abscissa and plotting a ray intensity on the ordinate.
Measurement apparatus: SmartLab System (manufactured by Rigaku Corporation)
Radiation source: Cukα1
Tube voltage: 45 kV
Tube current: 200 mA
Scanning range: 3 to 35°
Sampling width: 0.02°

Thermogravimetric analysis (TGA) analysis was performed using an EXSTAR TG/DTA 6200 R apparatus (manufactured by Seiko Instruments Inc. (current corporate name: Hitachi High-Tech Science Corporation)). 1 to 3 mg of a sample was put in an aluminum vessel. The analysis temperature ranged from 30°C to 350°C. The sample was analyzed at a temperature increase rate of 10°C/min under a nitrogen flow.

For HPLC analysis, measurement was performed at 225 nm with a PDA detector using H-Class System manufactured by Waters Corporation. For GC analysis, detection was performed with FID using GC 2010 manufactured by Shimadzu Corporation.

The products from the steps were evaluated by analysis methods shown in Table 1 below.

### [Table 1]

**Table 1**

| Measurement method | Column | Measurement conditions |
|---|---|---|
| GC | HP-5 (30 m, 0.320 mm, 0.25 µm) | 50°C (1 minute) → 20°C/min → 280°C (1.5 minutes) |
| | | Flow rate: 30 cm/sec |
| | | Detector: FID |
| HPLC Method A | Ascentis Express C18 (2.7 µm, 4.6 mm, 50 mm) | A: 0.05% TFA aqueous solution |
| | | B: 0.05% TFA acetonitrile solution |
| | | A:B = 95:5 (0 minute) - 77:23 (3.5 minutes) - 39:61 (8 minutes) - 0:100 (9.5 - 9.6 minutes) - 95:5 (9.7 minutes - 12 minutes) |
| | | Flow rate: 1 mL/min |
| | | Detection wavelength: 225 nm |
| HPLC Method B | Kinetex 2.6u XB-C18 100A (2.6 µm, 4.6 mmx 50 mm) | A: 0.05% TFA aqueous solution |
| | | B: 0.05% TFA acetonitrile solution |
| | | A:B = 70:30 (0 minute) - 5:95 (6 minutes) - 5:95 (8.5 minutes) - 70:30 (8.6 minutes) - 70:30 (10 minutes) |
| | | Flow rate: 1 mL/min |
| | | Detection wavelength: 225 nm |

### (Example 1-1) Production of 4-(trifluoromethyl)-2-(6-(trifluoromethyl)pyrimidin-4-yl)aniline

The compound was produced through the following production route.

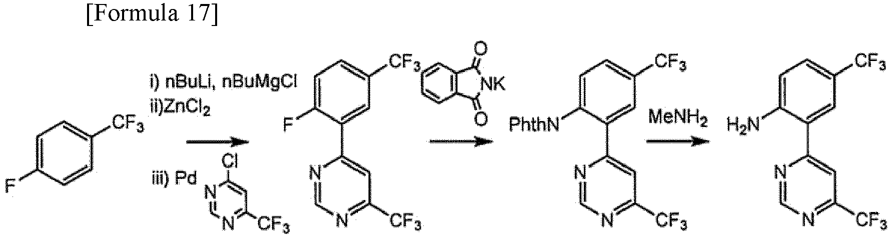

### [Step 1] Production of 4-(2-fluoro-5-(trifluoromethyl)phenyl)-6-(trifluoromethyl)pyrimidine

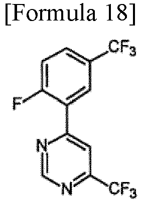

To a 3 L flask evacuated and back filled with nitrogen, 1-fluoro-(4-trifluoromethyl)benzene (150 g, 912 mmol) and tetrahydrofuran (700 mL) were added. The obtained solution was cooled to an internal temperature of -17.8°C with stirring, and *n-*butylmagnesium chloride (2.13 M, tetrahydrofuran solution, 100 mL, 213 mmol) was added dropwise over 12 minutes. Thereafter, to the reaction mixture, *n*-butyllithium (1.63 M, hexane solution, 298 mL, 486 mmol) was added dropwise over 1 hour and 21 minutes. The resulting mixture was stirred at an internal temperature between -10.5°C and -17.6°C for 4 hours and 30 minutes, and zinc chloride (2.03 M, 2-methyltetrahydrofuran solution, 300 mL, 608 mmol) was then added dropwise to the reaction mixture over 35 minutes. Thereafter, to the reaction mixture a 1,1'-bis(diphenylphosphino)ferrocene-palladium(II) dichloride dichloromethane complex (4.9717 g, 6.08 mmol) and 4-chloro-6-(trifluoromethyl)pyrimidine (111.033 g, 608 mmol) were added with tetrahydrofuran (45 mL) at an internal temperature of 0°C. The reaction mixture was heated to an internal temperature of 60°C over 30 minutes or more, and sampled 1 hour after the temperature reached 60°C. Disappearance of 4-chloro-6-(trifluoromethyl)pyrimidine was confirmed by GC analysis. Thereafter, the reaction mixture was cooled to an internal temperature of 25°C. To the reaction mixture was added citric acid (10% aqueous solution, 555 mL), the resulting mixture was stirred for 20 minutes, and then left standing, and the aqueous layer was discharged. To the reaction mixture was added EDTA-tetrasodium dihydrate (10% aqueous solution, 555 mL), the resulting mixture was stirred for 20 minutes, and then left standing, and the aqueous layer was discharged. To the reaction mixture was added water (555 mL), the resulting mixture was stirred for 10 minutes, and then left standing, and the aqueous layer was discharged. The obtained organic layer was concentrated under reduced pressure, toluene (555 mL) was added, and the resulting mixture was concentrated under reduced pressure again to obtain a crude product of 4-(2-fluoro-5-(trifluoromethyl)phenyl)-6-(trifluoromethyl)pyrimidine. The obtained crude product was used without further purification in a next step.
GC purity: 91.17%
Measurement method: GC, retention time: 7.4 minutes

### [Step 2] Production of 4-(trifluoromethyl)-2-(6-(trifluoromethyl)pyridin-4-yl)aniline

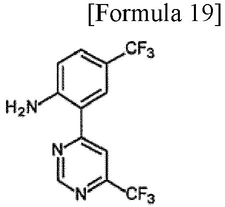

The crude product of 4-(2-fluoro-5-(trifluoromethyl)phenyl)-6-(trifluoromethyl)pyrimidine obtained in step 1 was transferred into a 5 L flask, and *N,N-*dimethylformamide (1.71 L) was added. To the above-described solution was added a solution obtained by adding *N*,*N*-dimethylformamide (190 mL) to potassium phthalimide (124.051 g, 670 mmol). Stirring of the reaction mixture was started, the external temperature was set to 110°C, and heating was started. 3 Hours after the internal temperature reached 100°C, the reaction mixture was sampled, and it was confirmed by HPLC analysis that the reaction conversion rate was 99.3%. Thereafter, the reaction mixture was cooled to an internal temperature of 53 °C. To the reaction mixture was added a methylamine aqueous solution (40% w/w. 567 mL). The reaction mixture was stirred at an internal temperature between 48°C and 53°C for 4 hours, and then sampled, and it was confirmed by HPLC analysis that the reaction conversion rate was 100%. A solution of *N-*acetyl-L-cysteine (9.9449 g, 60.9 mmol) in *N*,*N*-dimethylformamide (38 mL) was added, and the resulting mixture was stirred for 1 hour. To the reaction mixture, water (1134 mL) was added dropwise in six divided parts (189 mL × 6) over about 1 hour and 20 minutes. During addition of water, crystals were precipitated to form a suspension. After addition of water was completed, the mixture was stirred for 1 hour, and the reaction mixture was cooled to 25°C. The mixture was filtered, and the obtained crystals were washed twice with water (1980 mL × 2). The obtained wet powder was dried at an external temperature of 35°C under reduced pressure to obtain 4-(trifluoromethyl)-2-(6-(trifluoromethyl)pyrimidin-4-yl)aniline (169.62 g, yield in two steps: 90.7%).
HPLC purity: 96.42%
Measurement method: HPLC method A, retention time: 8.8 minutes
¹H-NMR (500 MHz, CDCl₃) δ: 6.71 (2H, brs), 6.83 (1H, d, J=9.0 Hz), 7.49 (1H, d, J=8.5 Hz), 7.95 (1H, s), 8.04 (1H, s), 9.34 (1H, s).

### (Example 1-2)

### [Step 1] Production of 4-(2-fluoro-5-(trifluoromethyl)phenyl)-6-(trifluoromethyl)pyrimidine

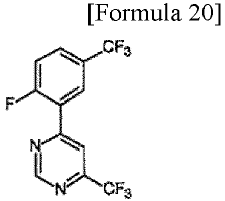

To a 3 L flask evacuated and back filled with nitrogen, 1-fluoro-(4-trifluoromethyl)benzene (150 g, 912 mmol) and tetrahydrofuran (720 mL) were added. This solution was cooled to an internal temperature of -20.3°C with stirring, and *n-*butylmagnesium chloride (2.13 M, tetrahydrofuran solution, 100 mL, 213 mmol) was added dropwise over 28 minutes. Subsequently, to the reaction mixture, n-butyllithium (1.63 M, hexane solution, 299 mL, 487 mmol) was added dropwise over 47 minutes, the inside of the dropping funnel was then washed with tetrahydrofuran (30 mL), and the washing liquid was added to the reaction mixture. The reaction mixture was stirred at an internal temperature between -8.6°C and -10.2°C for 4 hours, and then cooled to an internal temperature of - 20.1°C, and zinc chloride (1.0 M tetrahydrofuran solution, 609 mL, 609 mmol) was added dropwise over 61 minutes. The reaction mixture was heated to an internal temperature of 0°C, and stirred for 17 minutes, and a 1,1'-bis(diphenylphosphino)ferrocene-palladium(II) dichloride complex (4.98 g, 6.09 mmol) and 4-chloro-6-(trifluoromethyl)pyrimidine (111.084 g, 609 mmol) were then added to the reaction mixture. The reaction mixture was heated to an internal temperature of 60°C over 25 minutes, stirred at this temperature for 1 hour, and then sampled, and a part of the reaction mixture was analyzed by GC analysis to confirm the disappearance of 4-chloro-6-(trifluoromethyl)pyrimidine. The reaction mixture was cooled to an internal temperature of 10°C or lower, 1 M hydrochloric acid (750 mL) and toluene (750 mL) were added, the resulting mixture was stirred for 10 minutes, and the aqueous layer was then discharged. Subsequently, 1 M hydrochloric acid (300 mL) was added to the organic layer, the resulting mixture was stirred for 10 minutes, and the aqueous layer was then discharged. To the organic layer, a 10% *N*-(2-hydroxyethyl)ethylenediamine-*N*,*N*',*N*'-trisodium triacetate dihydrate aqueous solution (750 mL) was added, and the resulting mixture was stirred for 30 minutes, and the aqueous layer was then discharged. A 10% sodium chloride aqueous solution (750 mL) was added to the organic layer, the resulting mixture was stirred for 10 minutes, and the aqueous layer was then discharged. The obtained organic layer was concentrated under reduced pressure to obtain a crude product of 4-(2-trifluoro-5-(trifluoromethyl)phenyl)-6-(trifluoromethyl)pyrimidine. The obtained crude product was used without further purification in a next step.
GC purity: 94.35% (calculated only from the peaks after a retention time of 3.1 minutes)
Measurement method: GC, retention time: 7.4 minutes

### [Step 2] Production of 4-(trifluoromethyl)-2-(6-(trifluoromethyl)pyridin-4-yl)aniline

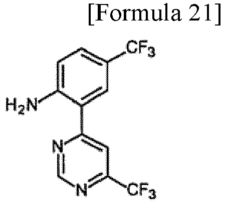

The crude product of 4-(2-trifluoro-5-(trifluoromethyl)phenyl)-6-(trifluoromethyl)pyrimidine obtained in step 1 of Example 1-2 was transferred into a 5 L flask, and *N*,*N*-dimethylformamide (1.5 L) was added. To this solution, potassium phthalimide (124.022 g, 670 mmol) was added with *N,N*-dimethylformamide (378 mL). While the reaction mixture was stirred, the reaction vessel external temperature was set to 103°C, and heating was started. 3 Hours after the internal temperature reached 100°C, the reaction mixture was sampled, and it was confirmed by HPLC analysis that the reaction conversion rate was 98.6%. The reaction mixture was further stirred at 100°C for 3 hours, the reaction mixture was then sampled again, and it was confirmed by HPLC analysis that the reaction conversion rate was 99.9%. Thereafter, the reaction mixture was cooled to an internal temperature of 50°C. A methylamine aqueous solution (40% w/w, 567 mL) was added to the reaction mixture, the resulting mixture was stirred at an internal temperature of 50°C for 3 hours, the reaction mixture was then sampled, and it was confirmed by HPLC analysis that the reaction conversion rate was 100%. To the reaction mixture, *N-*acetylcysteine (9.9447 g, 60.9 mmol) was added with *N,N-*dimethylformamide (18.9 mL), and the resulting mixture was stirred for 1 hour. To the reaction mixture, water (1134 mL) was added dropwise in six divided parts (189 mL × 6) over about 1 hour and 20 minutes. During dropwise addition of water, crystals were precipitated to form a suspension. After dropwise addition of water was completed, the mixture was stirred for 1 hour, and then cooled to 25°C. The suspension was filtered, and the obtained crystals were washed twice with water (1890 mL × 2). The obtained wet powder was dried at an external temperature between 30 and 40°C under reduced pressure to obtain 4-(trifluoromethyl)-2-(6-(trifluoromethyl)pyrimidin-4-yl)aniline (169.73 g, yield in two steps: 90.8%).
HPLC purity: 96.95%
Measurement method: HPLC method A, retention time: 8.8 minutes

### (Reference Example 1) Production of 4-(trifluoromethyl)-2-(6-(trifluoromethyl)pyrimidin-4-yl)aniline (conventional method)

The compound was produced through the following production route.

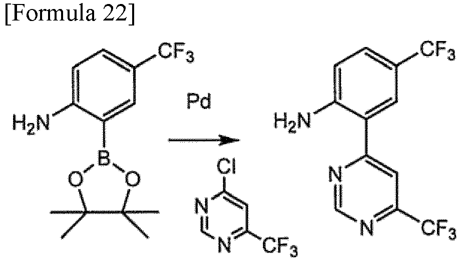

To a reaction vessel, a *N*,*N*-dimethylacetamide solution of 2-(4,4,5,5-tetramethyl-1,3,2-dioxaborolan-2-yl)-4-(trifluoromethyl)aniline (manufactured by Boron Molecular Inc. (catalog number: BM1088), 30.0 kg) (86.2 kg) in *N*,*N*-dimethylacetamide (22.2 L) were added under a nitrogen flow. Subsequently, 4-chloro-6-(trifluoromethyl)pyrimidine (20.6 kg) and *N*,*N*-dimethylacetamide (38.2 L) were added, a solution of 1,1'-bis(diphenylphosphino)ferrocene-palladium(II) dichloride dichloromethane complex (0.408 kg) in *N*,*N*-dimethylacetamide (166 L) and water (9.55 L) were further added, and the reaction vessel was then degassed under reduced pressure for 15 minutes, and then evacuated and back filled with nitrogen. A tripotassium phosphate aqueous solution prepared by adding water (38.2 L) to tripotassium phosphate (25.3 kg) was added under nitrogen flow while keeping internal temperature under 50°C. Thereafter, the reaction vessel was then degassed under reduced pressure for 5 minutes, and then evacuated and back filled with nitrogen. The reaction mixture was stirred in an internal temperature between 50 and 60°C for 2 hours, a solution prepared by adding *N*,*N*-dimethylacetamide (95.5 L) to *N*-acetyl-L-cysteine (1.63 kg) was then added, and the resulting mixture was further stirred in an internal temperature between 54.4 and 57.6°C for 1 hour. To the reaction mixture was added water (28.7 L) in ten divided parts in an internal temperature between 52.0 and 56.4°C. The reaction mixture was stirred in an internal temperature between 55.8 and 56.3°C for 30 minutes, then cooled to 25°C, and further stirred for 19 hours and 45 minutes. The obtained precipitate was collected by filtration, and then washed twice with water (287 L). The wet powder was dried at 40°C under a reduced pressure between 0.08 and 0.10 MPa for 70 hours to obtain 31.52 kg of the title compound (yield: 98.2%). The ¹H-NMR spectral data and the retention time in HPLC analysis for the obtained compound matched those from Example 1.

Table 2 shows comparison between Example 1-2 and Reference Example 1. The purity of the compound was calculated by HPLC analysis.

### [Table 2]

**Table 2:**

| | Example 1-2 | Reference Example 1 |
|---|---|---|
| Number of steps | 2 steps (3 reactions) | 1 step (1 reaction) |
| Yield | 90.8% (150 g scale, 2 steps) | 95-98% (30 kg scale) |
| Purity | 96.95% | 99.88% |

Example 1-2 was found to give a target product having a purity equivalent to that in Reference Example 1.

### (Reference Example 2) Production of 1-(2-chloroethoxy)-2,3-difluorobenzene

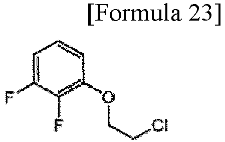

Using 2, 3-difluorophenol and 1,2-dichloroethane, the title compound was produced in accordance with a method described in a document (Williamson, AW et al., J. Chem. Soc. 1852, 106, 229-239).

### (Reference Example 3) Production of 2,3-difluoro-4-(2-((2-methoxyethyl)(methyl)amino)ethoxy)benzaldehyde

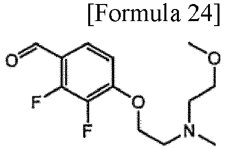

Using the 1-(2-chloroethoxy)-2,3-difluorobenzene obtained in Reference Example 2, the title compound was produced through the following production route described in International Publication No. WO 2014/142273.

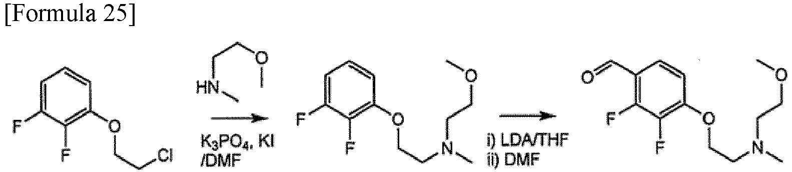

### (Reference Example 4) Production of methyl (E)-1-(2-(2,3-difluoro-4-(2-((2-methoxyethyl)(methyl)amino)ethoxy)benzilidene)-1-methylhydradinyl)cyclopentane-1-carboxylate oxalate

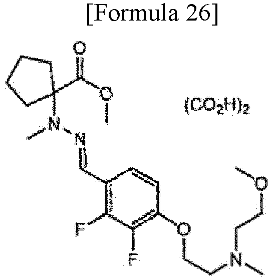

### [Step 1]

Using methyl 1-(methylamino)cyclopentane-1-carboxylate as a starting material and the 2,3-difluoro-4-(2-((2-methoxyethyl)(methyl)amino)ethoxy)benzaldehyde obtained in Reference Example 3, the title compound was produced through the following production route described in International Publication No. WO 2014/142273.

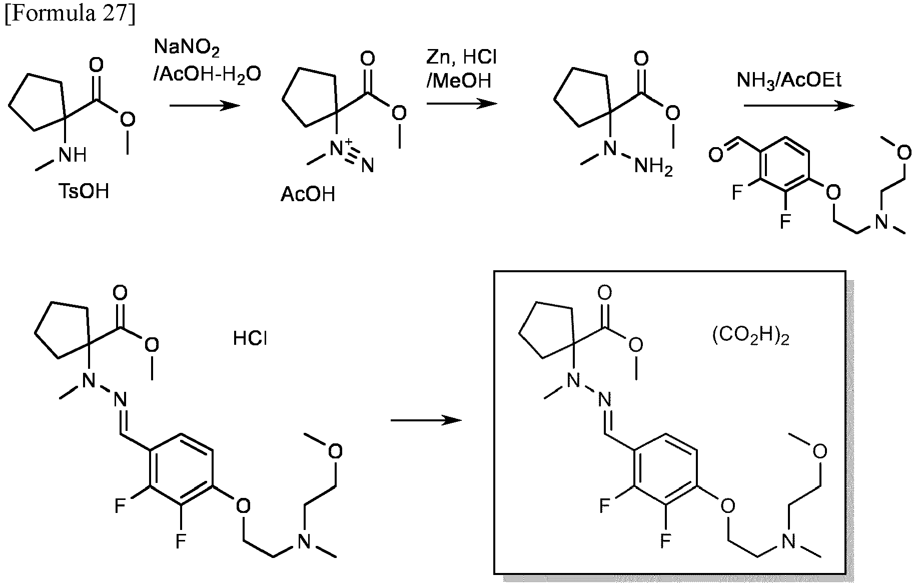

### [Step 2]

Methyl (E)-1-(2-(2,3 -difluoro-4-(2-((2-methoxyethyl)(methyl)amino)ethoxy)benzilidene)-1-methylhydradinyl)cyclopentane-1 - carboxylate hydrochloride (60.0 g, 129 mmol) and toluene (360 mL) were added to a reaction vessel in a nitrogen atmosphere to form a suspension. The obtained slurry was cooled, and a sodium hydrogen carbonate aqueous solution (prepared by dissolving 21.7 g of sodium hydrogen carbonate in 300 mL of water) was added dropwise while maintaining an internal temperature of -5°C. The resulting mixture was heated to room temperature, and stirred for 30 minutes. The organic layer was separated, the obtained aqueous layer was extracted with toluene (60 mL), and the combined organic layers were concentrated to obtain a toluene solution of (E)-1-(2-(2,3-difluoro-4-(2-((2-methoxyethyl)(methyl)amino)ethoxy)benzilidene)-1-methylhydradinyl)cyclopentane-1 - carboxylate (free form) (131.9 g). To this solution, toluene was added to total volume of 330 mL, and the resulting mixture was stored overnight at room temperature. After 13 hours, the reaction vessel external temperature was set to 50°C, a methanol solution of oxalic acid (prepared by dissolving 11.6 g of oxalic acid in 27.6 mL of methanol) was added at an internal temperature of 48°C, and the resulting mixture was stirred for 20 minutes. The reaction vessel external temperature was set to 25°C, and *n*-heptane (138 mL) was added to the obtained solution. The reaction vessel external temperature was set to 0°C, and the reaction mixture was stirred at an internal temperature of 1°C or lower for 1.5 hours. The precipitated solid was collected by filtration, and the obtained wet powder was washed with toluene (166 mL). The wet powder was dried at an external temperature of 40°C under reduced pressure to obtain the target product (56.7 g) (yield: 85%).
HPLC purity: 99.52%
Measurement method: HPLC method A, retention time: 6.7 minutes
¹H-NMR (DMSO-D₆) δ: 7.45-7.41 (1H, m), 7.27 (1H, s), 7.06 (1H, t, J=8.2 Hz), 4.42 (2H, t, J=5.3 Hz), 3.65 (2H, t, J=5.3 Hz), 3.61 (3H, s), 3.42 (2H, t, J=5.0 Hz), 3.29 (3H, s), 3.22 (2H, t, J=5.3 Hz), 2.85 (3H, s), 2.76 (3H, s), 2.28-2.23 (2H, m), 2.17-2.11 (2H, m), 1.73-1.67 (4H, m).

### (Example 2) Production of methyl 1-(2-(2,3-difluoro-4-(2-((2-methoxyethyl)(methyl)amino)ethoxy)benzyl)-1-methylhydradinyl)cyclopentane-1-carboxylate

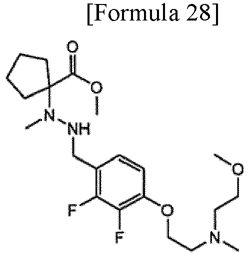

Under nitrogen atmosphere, toluene (375 ml) was added to methyl (E)-1-(2-(2,3-difluoro-4-(2-((2-methoxyethyl)(methyl)amino)ethoxy)benzilidene)-1-methylhydradinyl)cyclopentane-1-carboxylate oxalate (50.0 g, 97 mmol) obtained in Reference Example 4 and 2-picoline borane (8.7 g, 77 mmol) to afford a suspension. To this suspension, an oxalic acid aqueous solution (prepared by dissolving 17.4 g of oxalic acid in 235 ml of water) was added dropwise while maintaining the internal temperature of 25°C or lower. Thereafter, the resulting mixture was stirred for 8.5 hours. The organic layer was discarded, toluene (375 mL) was added to the aqueous layer, and the resulting mixture was stored overnight at room temperature. After 12 hours, a potassium hydroxide aqueous solution (prepared by dissolving 39.5 g of potassium hydroxide in 150 mL of water) was added dropwise while maintaining the internal temperature of 0°C or lower. The resulting mixture was stirred at 20°C for 30 minutes, and the aqueous layer was then discarded. A sodium chloride aqueous solution (prepared by dissolving 30.1 g of sodium chloride in 150 mL of water) was added to the organic layer, and the resulting mixture was stirred for 15 minutes. The aqueous layer was discharged, and the organic layer was concentrated to obtain a toluene solution of the target compound. To the obtained solution was added ethyl acetate (290 mL), and the resulting mixture was stored overnight at room temperature.
HPLC purity: 97.91%
Measurement method: HPLC method A, retention time: 4.3 minutes

### (Example 3) Production of 3-oxo-3-((4-(trifluoromethyl)-2-(6-(trifluoromethyl)pyrimidin-4-yl)phenyl)amino)propanoic acid

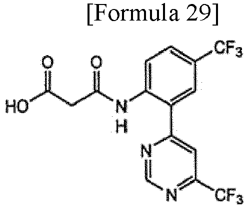

To a reaction vessel, the 4-(trifluoromethyl)-2-(6-(trifluoromethyl)pyrimidin-4-yl)aniline (2.0 g, 6.5 mmol) obtained in Example 1-2 and Meldrum's acid (0.94 g, 6.5 mol) were added under nitrogen atmosphere, toluene (10 mL) and n-heptane (10 mL) were added subsequently to afford a suspension. The reaction vessel external temperature was set to 110°C, and the reaction mixture was heated to an internal temperature of 100°C, and stirred for 5 hours. Completion of the reaction was confirmed by HPLC, and the reaction mixture was then cooled to an internal temperature of 25°C, and further stirred for 2 hours. The precipitated crystals were filtered, and then washed with a mixed solution prepared from toluene (4 mL) and n-heptane (4 mL). The wet powder was dried at an external temperature of 35°C under reduced pressure to obtain the target product (2.1 g) (yield: 80%).
HPLC purity: 99.67%
Measurement method: HPLC method A, retention time: 7.5 minutes
¹H-NMR (DMSO-D₆) δ: 11.01 (1H, s), 9.53 (1H, s), 8.47 (1H, s), 8.25 (1H, d, J=8.6 Hz), 8.22-8.21 (1H, m), 7.96-7.94 (1H, m), 3.42 (2H, s).

### (Reference Example 5) Production of 3-oxo-3-((4-(trifluoromethyl)-2-(6-(trifluoromethyl)pyrimidin-4-yl)phenyl)amino)propanoic acid (conventional method)

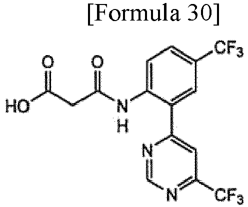

To a reaction vessel, the 4-(trifluoromethyl)-2-(6-(trifluoromethyl)pyrimidin-4-yl)aniline (54.88 kg) obtained in Reference Example 1 and Meldrum's acid (25.76 kg) were added under nitrogen atmosphere, toluene (274 L) and n-heptane (274 L) were further added, to afford a suspension. The reaction mixture was heated to an internal temperature of 90°C or higher, and stirred for 5 hours. Completion of the reaction was confirmed by HPLC, and the reaction mixture was then cooled to an internal temperature of 25°C or lower, and further stirred at an internal temperature between 15 and 25°C for 1 hour or more. The precipitated crystals were collected by filtration, and then washed with a mixed solution prepared from toluene (110 L) and n-heptane (110 L). The wet powder was dried at an external temperature of 40°C under reduced pressure to obtain the target product (61.82 kg) (yield: 88%).
HPLC purity: 99.9%
Measurement method: HPLC method A, retention time: 7.5 minutes

Comparison between Example 3 and Reference Example 5 is shown below. The purity of the compound was calculated by HPLC analysis.

**[Table 3]**

| | Example 3 | Reference Example 5 |
|---|---|---|
| Yield (%) | 80% | 88% |
| Purity (%) | 99.67% | 99.9% |

Example 3 was found to give a target product having a purity equivalent to that in Reference Example 5.

### (Example 4-1) Production of methyl 1-((2-(2,3-difluoro-4-(2-((2-methoxyethyl) (methyl)amino)ethoxy)phenyl)methyl)-1-methyl-2-(3-oxo-3-((4-(trifluoromethyl)-2-(6-(trifluoromethyl)pyrimidin-4-yl)phenyl)amino)propanoyl)hydradinyl)cyclopentane-1-carboxylate

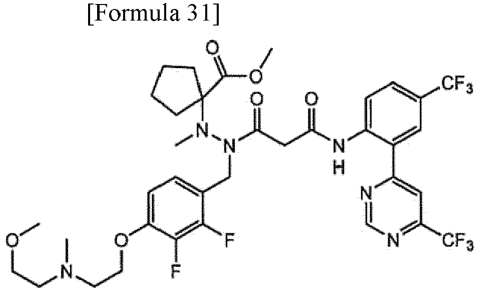

To a reaction vessel, under nitrogen atmosphere, were added a solution of the methyl 1-(2-(2,3-difluoro-4-(2-((2-methoxyethyl) (methyl)amino)ethoxy)benzyl)-1-methylhydradinyl)cyclopentane-1-carboxylate obtained in Example 2 and a *N,N-*dimethylformamide (145 mL) solution of the 3-oxo-3-((4-(trifluoromethyl)2-(6-(trifluoromethyl)pyrimidin-4-yl)phenyl)amino)propionic acid (39.9 g, 101 mmol) obtained in Reference Example 5. To the obtained solution, pyridine (41.5 mL, 513 mmol) was added dropwise while maintaining the internal temperature at -5°C. Subsequently, a 50% ethyl acetate solution of propylphosphonic anhydride (43.0 g, 135 mmol) was added dropwise to the reaction mixture while the internal temperature was maintained at -5°C. The reaction mixture was stirred at 5°C for 15 minutes, 3-oxo-3-((4-(trifluoromethyl)-2-(6-(trifluoromethyl)pyrimidin-4-yl)phenyl)amino)propionic acid (1.9 g, 4.8 mmol) was then added, and the resulting mixture was further stirred for 3.5 hours. A sodium chloride aqueous solution (prepared by dissolving 12.5 g of sodium chloride in 124 mL of water) was added to the reaction mixture at 25°C, and the resulting mixture was stirred for 20 minutes. The aqueous layer was discarded, a sodium chloride aqueous solution (prepared by dissolving 12.5 g of sodium chloride in 124 mL of water) was further added to the organic layer, and the resulting mixture was stirred for 20 minutes. The aqueous layer was discarded, a dipotassium hydrogen phosphate aqueous solution (prepared by dissolving 45.7 g of dipotassium hydrogen phosphate in 249 mL of water) was then added to the organic layer, the resulting mixture was stirred for 15 minutes, and the aqueous layer was then discharged. The organic layer was concentrated, the precipitated inorganic salt was removed by filtration with a Kiriyama funnel, and the filtrate was stored overnight at room temperature. After 13 hours, the filtrate was concentrated to dryness, and 2-propanol (116 mL) was added to the residue. The obtained solution was stirred for 10 minutes, and 2-propanol (382 mL) was then further added. The reaction vessel external temperature was set to 35°C, the reaction mixture was stirred at an internal temperature between 31 °C and 33°C for 1 hour, and the reaction vessel external temperature was lowered to 10°C over 1.25 hours. The reaction mixture was stirred at an internal temperature between 12°C and 14°C for 30 minutes, and the reaction vessel external temperature was increased to 35°C. The reaction mixture was stirred for 30 minutes while the internal temperature was maintained between 30°C and 35°C, and the reaction vessel external temperature was then lowered to 0°C over 1.75 hours. The reaction mixture was cooled to an internal temperature of 5°Cor lower, and the precipitated solid was then collected by filtration. The obtained wet powder was washed with 2-propanol (207 mL) pre-cooled to 0°C. The wet powder was dried at an external temperature between 35 and 40°C under reduced pressure to obtain the target product (65.7 g) (yield by two steps: 84%).
HPLC purity: 99.49%
Measurement conditions: HPLC method B, HPLC retention time: 3.9 minutes
¹H NMR (500 MHz, DMSO-D₆) δ: 11.15 (1H, s), 9.51 (1H, d, J=0.9 Hz), 8.54 (1H, d, J=1.4 Hz), 8.32 (1H, d, J=8.6 Hz), 8.25 (1H, d, J=1.4 Hz), 7.97-7.95 (1H, m), 7.00-6.98 (1H, m), 6.93-6.91 (1H, m), 4.70 (1H, d, J=16.3 Hz), 4.41 (1H, d, J=16.3 Hz), 4.14-4.08 (2H, m), 3.96 (1H, d, J=15.4 Hz), 3.69 (1H, d, J=15.4 Hz), 3.61 (3H, s), 3.40 (2H, t, J=5.9 Hz), 3.22 (3H, s), 2.76 (2H, t, J=5.7 Hz), 2.59-2.57 (5H, m), 2.27 (3H, s), 2.04-1.97 (2H, m), 1.85-1.48 (6H, m).

### (Example 4-2) Synthesis of methyl 1-(2-((2,3-difluoro-4-(2-((2-methoxyethyl) (methyl)amino)ethoxy)phenyl)methyl)-1-methyl-2-(3-oxo-3-((4-(trifluoromethyl)2-(6-(trifluoromethyl)pyrimidin-4-yl)phenyl)amino)propanoyl)hydradinyl)cyclopentane-1-carboxylate using (EtO)₂POCl

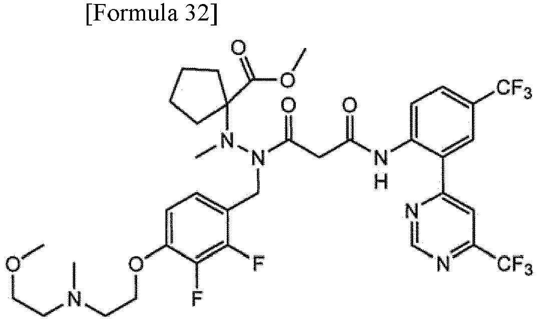

A *N*,*N*-dimethylformamide solution (1.0 mL) of methyl 1-(2-(2,3-difluoro-4-(2-((2-methoxyethyl) (methyl)amino)ethoxy)benzyl)-1-methylhydradinyl)cyclopentane-1-carboxylate (0.109 g, 0.254 mmol) and 3-oxo-3-((4-(trifluoromethyl)2-(6-(trifluoromethyl)pyrimidin-4-yl)phenyl)amino)propionic acid (0.101 g, 0.257 mmol) were mixed, and the reaction vessel was then evacuated and back filled with nitrogen. Pyridine (0.031 mL, 0.381 mmol) and (EtO)₂POCl (0.073 mL, 0.509 mmol) were added in this order to keep the internal temperature at -10°C. The reaction mixture was stirred at an internal temperature of -10°C for 1.5 hours, and the conversion rate of the target product was determined from a HPLC area ratio.
Production rate: 76.49%
Measurement conditions: HPLC method A, retention time: 8.4 minutes

### (Example 5) Production of 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl1-6,7-diazaspiro[4,5]dec-9-ene-9-carboxamide

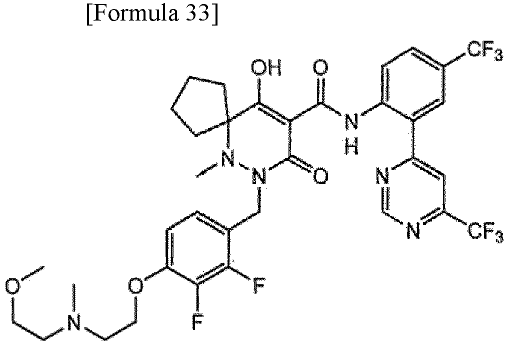

Methyl 1-(2-(2,3-difluoro-4-(2-((2-methoxyethyl) (methyl)amino)ethoxy)benzyl)-1-methyl-2-(3-oxo-3-((4-(trifluoromethyl)-2-(6-(trifluoromethyl)pyrimidin-4-yl)phenyl)amino)propanoyl)hydrazinyl)cyclopentane-1-carboxylate (34 kg, 42 mol), potassium carbonate (11.7 kg, 85 mol) and ethanol (268 kg) were added to a reaction vessel to afford a suspension, and stirring and heating were started. The reaction mixture was stirred for 2 hours after reaching reflux and it was confirmed by HPLC analysis that the reaction conversion rate was 100%. The obtained suspension was concentrated under reduced pressure, and ethyl acetate (170 L) was added. To the reaction mixture was added a 1 M hydrochloric acid aqueous solution (85 kg), the resulting mixture was stirred for 15 minutes, and then left standing, and the aqueous layer was discharged. To the reaction mixture was added a 10% potassium dihydrogen phosphate aqueous solution (170 kg), the resulting mixture was stirred for 15 minutes, and then left standing, and the aqueous layer was discharged. To the reaction mixture was added a 10% sodium chloride aqueous solution (170 kg), the resulting mixture was stirred for 15 minutes, and then left standing, and the aqueous layer was discharged. The organic layer was concentrated under reduced pressure, ethyl acetate (80 L) was added to the obtained concentrate, and the resulting mixture was filtered. After the filtration, ethyl acetate (80 L) was used for washing the vessel and pipes. The filtrate was concentrated under reduced pressure again, and it was confirmed by ¹H-NMR analysis that the molar ratio of ethyl acetate to a target product was 3 or less. To the obtained crude product of the title compound were added ethanol (52 kg) and n-heptane (52 kg).
HPLC purity: 99.24%
Measurement conditions: HPLC method A, retention time: 9.4 minutes

### (Example 6) Production of 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl]-6,7-diazaspiro[4,5]dec-9-ene-9-carboxamide p-toluenesulfonate

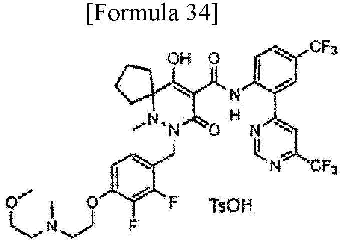

Under nitrogen atmosphere, a mixed solvent (52 kg of ethanol and 56 kg of *n-*heptane) was added to the 7-[[2,3-difluoro-4-[2-[2-methoxyethyl(methyl)amino]ethoxy]phenyl]methyl]-10-hydroxy-6-methyl-8-oxo-N [4-(trifluoromethyl)-2-[6-(trifluoromethyl)pyrimidin-4-yl]phenyl]-6,7-diazaspiro[4,5]dec-9-ene-9-carboxamide (33 kg, 42 mol) obtained in Example 5, and the obtained solution was stirred at an internal temperature of 25°C. *p*-Toluenesulfonic acid monohydrate (8 kg, 42 mol) dissolved in ethanol (13 kg) was added. Crystals of the title compound (0.03 kg, 0.035 mol) (prepared by a method disclosed Non Patent Literature 10) was suspended in *n*-heptane (0.5 L), and added as seed crystals, the vessel for preparing the suspension was then washed with *n*-heptane (0.5 L), and the washing mixture was added. The resulting mixture was stirred for 1 hour, precipitation of crystals was confirmed, *n*-heptane (56 kg) was then added, and the mixture was heated to an internal temperature of 33°C. The resulting mixture was stirred at an internal temperature of 30°C or higher for 30 minutes or more, then set the external temperature to 15°C, and stirred at an internal temperature of 25°C or less for 30 minutes or more. Subsequently, *n*-heptane (56 kg) was added, and the resulting mixture was stirred for 1 hour or more. The obtained suspension was filtered, and the wet powder was washed twice with *n*-heptane (67 kg). The wet powder was dried at an external temperature of 40°C under reduced pressure to obtain the title product (36.7 kg) (yield: 89%).
HPLC purity: 99.92%
Measurement conditions: HPLC method A, retention time: 9.4 minutes
¹H-NMR (CDCl₃) δ: 16.60 (1H, s), 12.82 (1H, s), 11.40 (1H, brs), 9.60 (1H, s), 8.49 (1H, d, J=8.6 Hz), 7.95 (1H, s), 7.90 (1H, d, J=1.5 Hz), 7.79 (1H, dd, J=8.6, 1.5 Hz), 7.75 (2H, d, J=8.0 Hz), 7.16 (2H, d, J=8.0 Hz), 7.06 (1H, dd, J=7.6, 7.7 Hz), 6.73 (1H, dd, J=7.6, 7.8), 5.05 (1H, d, J=14.3 Hz), 4.56 (2H, m), 4.21 (1H, d, J=14.3 Hz), 3.86 (2H, m), 3.80 (1H, m), 3.60 (1H, m), 3.54 (1H, m), 3.40 (1H, m), 3.36 (3H, s), 3.08 (3H, d, J=4.3 Hz), 2.48 (3H, s), 2.35 (3H, s), 2.16 (1H, m), 1.74 (2H, m), 1.57 (1H, m), 1.53 (1H, m), 1.48 (2H, m), 1.31 (1H, m).

### (Example 7) Production of 7-[[2,3-difluoro-4-[2-[2-methoxyethyl(methyl)amino]ethoxy]phenyl]methyl]-10-hydroxy-6-methyl-8-oxo-A-[4-(trifluoromethyl)-2-[6-(trifluoromethyl)pyrimidin-4-yl]phenyl]-6,7-diazaspiro[4,5]dec-9-ene-9-carboxamide p-toluenesulfonate

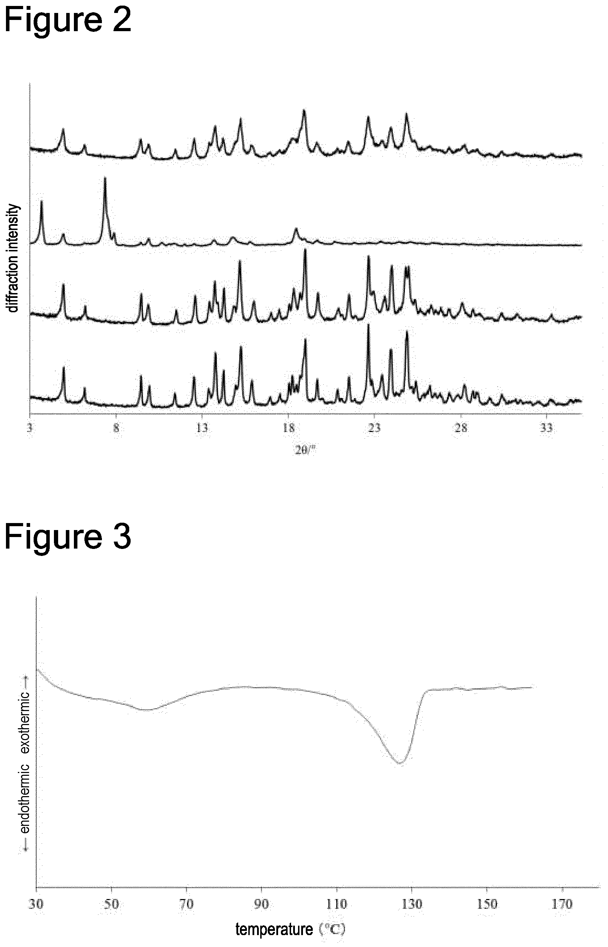

An ethanol *p*-toluenesulfonate solution (0.5 M, 0.935 mL) was added to 7-[[2,3-difluoro-4-[2-[2-methoxyethyl(methyl)amino]ethoxy]phenyl]methyl]-10-hydroxy-6-methyl-8-oxo-*N*-[4-(trifluoromethyl)-2-[6-(trifluoromethyl)pyrimidin-4-yl]phenyl]-6,7-diazaspiro[4,5]dec-9-ene-9-carboxamide (311.6 mg), and then the solids were dissolved at 60°C. Thereafter, a seed crystals were added with stirring at room temperature, and after 30 minutes, many crystal precipitates were observed. After 24 hours, the obtained crystals were collected by filtration, and X-ray powder diffraction measurement of the wet powder was performed. Figure 2 shows the measurement results. Further, the wet powder was placed at room temperature for 1 hour, and X-ray powder diffraction measurement of the dry powder was performed. While the latter powder was the same as a known crystal form, the former powder had a different X-ray powder diffraction pattern, and it was confirmed that in the drying step, crystal transition occurred rapidly. The yield was 298.0 mg.

In Figure 2, the X-ray powder diffraction patterns of a crystal obtained by a method disclosed in a document (Non Patent Document 10, Technique No. 2017-501666) (acquired using ethyl acetate), a crystal obtained by a method disclosed in the foregoing document (acquired using acetone), the wet powder obtained in Example 7, and the dry powder obtained in Example 7 are shown in this order from the bottom.

2θ Values of wet powder: the powder had diffraction peaks at 3.7°, 5.0°, 7.4°, 7.9°, 14.8° and 18.4° (± 0.2°).

2θ Values of dry powder: the powder had diffraction peaks at 4.9°, 9.4°, 9.9°, 15.2°, 15.9°, 18.9° and 22.7° (± 0.2°).

Thermogravimetric analysis of the dry powder of Example 7 was performed.
Figure 3 shows the measurement results. Peaks associated with melting appear at 112.6°C (extrapolation point) and 126.6°C (peak top).

### (Example 8) Production of 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl]-6,7-diazaspiro[4,5]dec-9-ene-9-carboxamide p-toluenesulfonate

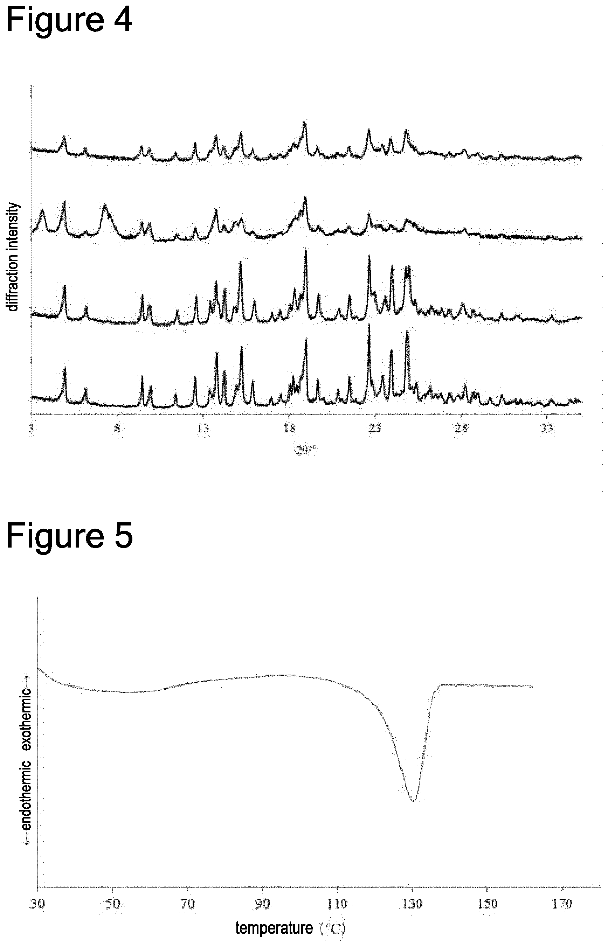

An ethanol *p*-toluenesulfonate solution (0.5 M, 0.936 mL) was added to 7-[[2,3-difluoro-4-[2-[2-methoxyethyl(methyl)amino]ethoxy]phenyl]methyl]-10-hydroxy-6-methyl-8-oxo-*N*-[4-(trifluoromethyl)-2-[6-(trifluoromethyl)pyrimidin-4-yl]phenyl]-6,7-diazaspiro[4,5]dec-9-ene-9-carboxamide (311.9 mg), and then solids were dissolved at 60°C. Thereafter, a seed crystals and n-heptane (0.936 mL) were added with stirring at room temperature, and after 30 minutes, many crystal precipitates were observed. Further, n-heptane (1.871 mL) was added. After 24 hours, the obtained crystals were collected by filtration, and X-ray powder diffraction measurement of the wet powder was performed. Further, the wet powder was placed at room temperature for 1 hour, and X-ray diffraction measurement of the dry powder was performed. Figure 4 shows the measurement results. While the X-ray powder diffraction pattern of the dry powder was the same as that of Form I crystal, the X-ray powder diffraction pattern of the wet powder was different from the X-ray powder diffraction pattern of the dry powder, and it was confirmed that in the drying step, crystal transition occurred rapidly. The yield was 257.1 mg.

In Figure 4, the X-ray powder diffraction patterns of a crystal obtained by a method disclosed in a document (Non Patent Document 10, Technique No. 2017-501666) (acquired using ethyl acetate), a crystal obtained by a method disclosed in the foregoing document (acquired using acetone), the wet powder obtained in Example 8, and the dry powder obtained in Example 8 are shown in this order from the bottom.

2θ Values of wet powder: the powder had diffraction peaks at 3.7°, 4.9°, 7.3°, 7.7°, 14.2° and 18.7° (± 0.2°). 2θ Values of dry powder: the powder had diffraction peaks at 4.9°, 9.4°, 9.9°, 15.2°, 15.9°, 18.9° and 22.6° (± 0.2°).

Thermogravimetric analysis of the dry powder of Example 8 was performed. Figure 5 shows the analysis results.

Peaks associated with melting appear at 119.6°C (extrapolation point) and 130.2°C (peak top).

### INDUSTRIAL APPLICABILITY

The present invention provides a method for producing ap-toluenesulfonate of 7-(2,3-difluoro-4-(2-((2-methoxyethyl)(methyl)amino)ethoxy)benzyl)-10-hydroxy-6-methyl-*N-*(4-methyl-2-(6-methylpyrimidin-4-yl)phenyl)-8-oxo-6,7-diazaspiro[4,5]dec-9-ene-9-carboxamide which is useful as an active ingredient of a medicament. By using the production method of the present invention, a *p*-toluenesulfonate of 7-(2,3-difluoro-4-(2-((2-methoxyethyl)(methyl)amino)ethoxy)benzyl)-10-hydroxy-6-methyl-*N*-(4-methyl-2-(6-methylpyrimidin-4-yl)phenyl)-8-oxo-6,7-diazaspiro[4,5]dec-9-ene-9-carboxamide can be efficiently produced, and supplied.

## Claims

1. A method for producing crystalline powder of a *p*-toluenesulfonate of a compound of formula 1: 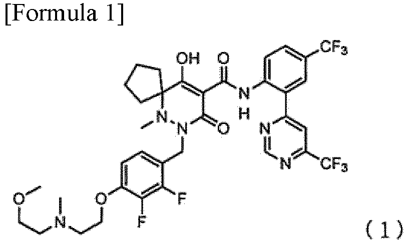 the method comprising:
converting the compound of formula 1 into the *p*-toluenesulfonate in a solvent and collecting a precipitated solid to obtain wet powder of the *p*-toluenesulfonate; and
subjecting the wet powder to drying conditions to obtain dry powder of a Form I crystal of the *p*-toluenesulfonate.

2. The method according to claim 1, comprising further adding a poor solvent to a mixture containing the solid before the collection.

3. The method according to claim 1 or 2, wherein the poor solvent is a solvent containing hexane or heptane; a solvent selected from hexane and heptane; or a mixed solvent containing hexane and heptane.

4. The method according to any one of claims 1 to 3, wherein the dry powder of the Form I crystal of the *p*-toluenesulfonate has an X-ray powder diffraction pattern including at least one selected from peaks at 4.9°, 9.4°, 9.9°, 15.2°, 15.8°, 18.9° and 22.6° (± 0.5°) as a diffraction angle 2θ in X-ray powder diffraction.

5. The method according to any one of claims 1 to 4, wherein the dry powder of the Form I crystal of the *p*-toluenesulfonate has an X-ray powder diffraction pattern including at least one selected from peaks at 4.9°, 9.4°, 15.8°, 18.9° and 22.6° (± 0.5°) as a diffraction angle 2θ in X-ray powder diffraction.

6. The method according to any one of claims 1 to 5, wherein the dry powder of the Form I crystal of the *p*-toluenesulfonate has an X-ray powder diffraction pattern including a peak at 15.8° (± 0.5°) as a diffraction angle 2θ in X-ray powder diffraction.

7. The method according to any one of claims 1 to 6, wherein the dry powder of the Form I crystal of the p-toluenesulfonate has an X-ray powder diffraction pattern including at least one selected from peaks at 4.9°, 9.4°, 9.9°, 15.2°, 15.8°, 18.9° and 22.6° (± 0.2°) as a diffraction angle 2θ in X-ray powder diffraction.

8. A method for producing a compound of formula 2: 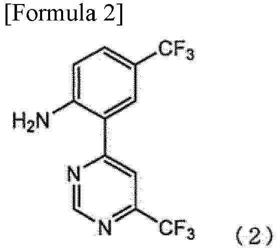 the method comprising:
treating a compound of formula 3: 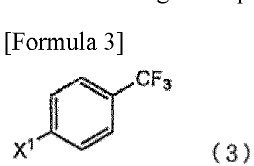
wherein X¹ is a leaving group,
with a C₁₋₆ alkylmagnesium halide, a C₁₋₆ alkyllithium and a zinc halide, and reacting the treated compound with a compound of formula 4: 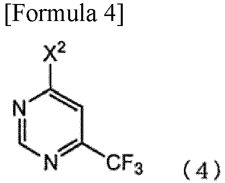
wherein X² is a leaving group,
in the presence of a palladium catalyst to obtain a compound of formula 5: 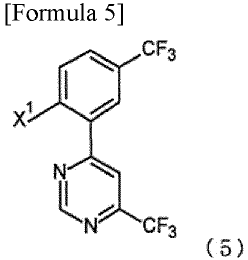 ; and
reacting the compound of formula 5 with an alkali metal salt of a dicarboxylic acid imide, followed by a decomposition reaction of the resulting imide compound to obtain a compound of formula 2.

9. The method according to claim 8, wherein X¹ is a halogen atom.

10. The method according to claim 8 or 9, wherein X² is a chlorine atom.

11. The method according to any one of claims 8 to 10, wherein the palladium catalyst is selected from the group consisting of a 1,1'-bis(diphenylphosphino)ferrocene-palladium(II) dichloride complex, a 1,1'-bis(diphenylphosphino)ferrocene-palladium(II) dichloride dichloromethane complex, bis(triphenylphosphine)palladium(II) dichloride, dichlorobis(tricyclohexylphosphine)palladium (II), [1,3-bis(2,6-diisopropylphenyl)imidazol-2-ylidene] (3-chloropyridyl)palladium(II) dichloride, (1,3-bis(2,6-diisopropylphenyl)imidazolidene) (3-chloropyridyl)palladium(II) dichloride and [1,3-bis(2,6-di-3-pentylphenyl)imidazol-2-ylidene] (3-chloropyridyl)palladium(II) dichloride.

12. The method according to any one of claims 8 to 11, wherein the alkali metal salt of a dicarboxylic acid imide is potassium phthalimide or sodium phthalimide.

13. A method for producing a compound of formula 1: 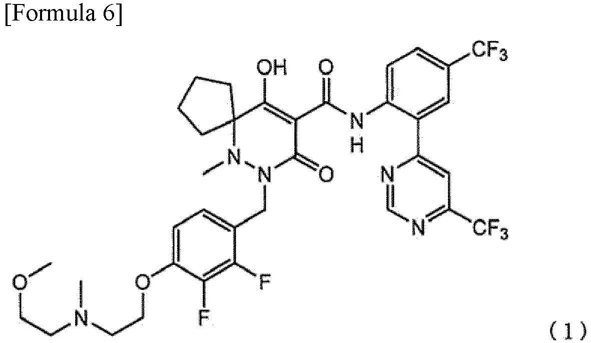 the method comprising:
1) preparing a compound of formula 2 by the method according to any one of claims 8 to 12;
2) reacting the compound of formula 2 with a malonic acid derivative to prepare a compound of formula 6: 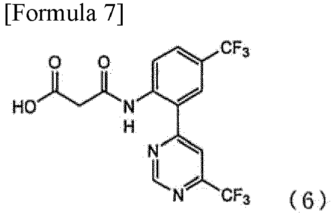 ;
3) reacting the compound of formula 6 with a compound of formula 7: 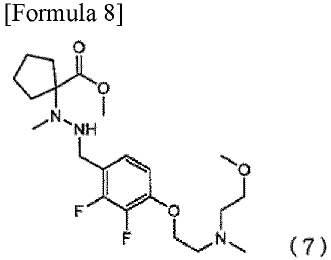 in the presence of a condensing agent to prepare a compound of formula 8: 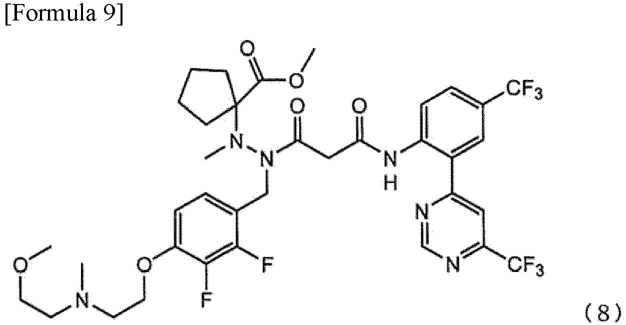 ; and
4) subjecting the compound of formula 8 to a cyclization reaction to prepare the compound of formula 1.

14. The method according to claim 13, wherein the malonic acid derivative is selected from the group consisting of Meldrum's acid, a dialkylmalonic acid and malonic acid.
